# EUROPEAN PATENT APPLICATION

(11) **EP 1 685 848 A1**
(43) Date of publication of application: **02.08.2006**
(21) Application number: 05075203.9
(22) Date of filing: 26.01.2005
(51) Int. Cl.: A61K 39/12, A61K 39/385

(54) **Oral vaccines for fish**

(71) Applicant: Plant Research International B.V., 6708 PB Wageningen (NL)
(72) Inventor: Florack, Dionisius Elisabeth Antonius, 6708 MD Wageningen (NL); Bosch, Hendrik Jan, 6708 NA Wageningen (NL)
(74) Representative: Winckels, Johannes Hubertus F.

(57) **Abstract**

The invention relates to the development, composition and production of mucosal (oral) vaccines for fish. More specifically, the invention relates to protein complexes for the delivery of antigens to and across mucosal surfaces of fish for the induction of an immune response, and to the production of said complexes in a host cell, preferably plants. Provided is the use of a protein complex comprising an antigen of interest fused to the B-subunit of *Vibrio cholerae* cholera toxin (CT-B), or *Escherichia coli* heat-labile enterotoxin (LT-B) for the manufacture of an oral fish vaccine. Also provided is fish feed comprising a protein complex of the invention.

## Description

The invention relates to the development, composition and production of mucosal (oral) vaccines for fish. More specifically, the invention relates to protein complexes for the delivery of antigens to and across mucosal surfaces of fish for the induction of an immune response, and to the production of said complexes in a host cell, preferably plants.

Infectious diseases are the main threat to European and world aquaculture. Intensification of aquaculture has led to an increasing number and frequency of infectious disease outbreaks, resulting in high economical losses and fish suffering. In addition, more fish species are being cultured, each with intrinsic infectious disease risks. Outbreaks often result in high mortality in small as well as in larger fish. Particularly viral diseases are an increasing problem (Leong and Fryer, 1993; Newman, 1993). Examples include Infectious Pancreatic Necrosis (IPN) caused by IPN virus (IPNV) affecting both salmonid and non-salmonid species; Viral Haemorrhagic Septicaemia (VHS) caused by VHS virus (VHSV) and most damaging to farmed rainbow trout (*Oncorhynchus mykiss*) and Spring Viraemia of Carp (SVC) caused by SVC virus (SVCV) and Koi Herpesvirus (KHV) affecting carp (*Cyprinus carpio*) but also koi. Other diseases of fish with high probability of occurrence and high economic consequences are Furunculosis, ISA, Hitra disease and SRS in salmon, ERM and Lactococcus in rainbow trout and Vibriosis and Pasteurellosis in sea bass and Vibriosis, Furunculosis and Hitra disease in atlantic cod.

The key to controlling most diseases in fish is identifying the controllable risk factors in disease prevention, rather than putting considerable efforts into eliminating the disease-causing pathogen. It often happens that when a disease occurs, conditions have been created that favour the pathogen over the fish. Whether a fish becomes diseased when a pathogen is present depends on factors that include fish health, water quality and temperature, stocking density, pathogen load, vaccination status, handling practices, uniformity of grade, and proximity of neighboring farms which may experience different disease threats. Of these, vaccination status offers aquaculture producers an effective way to lower both the risk of disease in their fish and their cost of production.

There are three common methods of vaccination: immersion, injection, and oral. These methods vary in terms of ease of administration, cost, stress on the fish, survival rates, dosage control, the amount of labour involved, and the duration of protection. Ultimately, the decision concerning which of these methods to use is based upon a combination of actual and perceived risk, age of the fish, the farmer's own risk-aversion, and return on investment.

It is generally accepted that injectable vaccines provide greater protection than immersion and oral vaccines because they allow for greater dosage control, which results in higher efficacy levels and a longer duration of protection. However, injectable vaccines tend to be more labour intensive, more expensive, and can cause damage to the fish if not administered with care. Furthermore, it precludes its use to small fish and it is expensive. Also, a number of side reactions can occur to either the immunising antigen or to the emulsifying reagent in which it is presented. Hence, injection vaccines if available, are of little use for mass vaccination of small fish under farm conditions.

Immersion vaccination is frequently used in fish farming but has the disadvantages that it is stressful for the fish and not completely protective. Thus, the most attractive method is oral vaccination which is relatively problem free.

Oral vaccine delivery has the major advantage that it enables the farmer to protect juvenile fish as soon as they start to feed. Advantageously, this usually coincides with the moment at which the young fish are most susceptible to attack and colonisation by pathogens. Oral vaccination also enables the farmer to administer booster doses to the fish whenever there is an increased risk of exposure to the pathogen. And last but not least, oral vaccination stimulates immune responses at the portal entrance of many pathogens. The major advantage of oral vaccines is that they enable the farmer to immunise fish with a minimum of stress and handling at mass scale and from the moment immune competent fish start to feed.

The development of practical oral vaccines for aquaculture remains the major elusive goal of the aquaculture industry worldwide. Oral vaccines that can be formulated in fish feed allow the antigen to be administered as soon as immune competent fish start to feed on pelleted feed. Major hurdles which to date have prevented the development of such oral vaccines are 1) the applied antigen (Ag) is often destroyed due to gastric acidity and protease activity present in the intestinal tract; 2) oral tolerance can be evoked and 3) the Ag does not necessarily enters the gut mucosa and consequently an immune response is not initiated.

Destruction of the Ag in the gut can be avoided by Ag encapsulation. For example, oral vaccination with Vibrio *anguillarum* bacterin antigen encapsulated in alginate microparticles (to protect the vaccine against degradation in the anterior part of the digestive tract) evoked systemic memory and induces mucosal immune responses in fish. (Joosten et al. (1997) Fish and Shellfish Immunology 7: 471). Furthermore, liposomes have attracted considerable interest as carriers and adjuvants for developing oral vaccines. The multilayer phospholipid vesicles protect the entrapped antigen from low pH and enzymatic attack until they reach their target sites (see for example Gregoriadis, Immun. Today 19990; 11:89). Oral vaccination with liposome-encapsulated antigens has been reported in fish. Irie et al. (2003) reported significant increases in anti-BSA antibodies in serum of carp *(Cyprinus carpio*) upon oral administration of liposome-entrapped bovine serum albumine (BSA) as model antigen.

In general however, the manufacture of these protected oral vaccine formulations is elaborate and expensive. They are therefore not suitable for large scale and cost-effective application, such as in aquaculture.

An object of the present invention is to provide an oral vaccine that allows for the delivery of antigens to fish mucosa which is cost-effective and which can be easily formulated in fish feed.

The invention provides the use of a protein complex comprising an antigen of interest fused to the mucosa-binding B-subunit of *Vibrio cholerae* cholera toxin (CT), or *Escherichia coli* heat-labile enterotoxin (LT) as an oral fish vaccine. In particular, it provides the use of such complex for the manufacture of an oral vaccine formulation for fish. It was surprisingly found that the problems of antigen destruction, antigen uptake as well as oral tolerance in fish can be overcome by the use of functional protein complexes that allow for the delivery of antigens to fish mucosa, promoting binding and uptake of the complex via mucosal cell surface receptors and the induction of immune responses to said antigen. The fusion protein complexes were shown to induce a specific immune response in fish that had been fed with feed pellets containing the protein complex. Surprisingly, antigen protection or encapsulation was not necessary.

In mammals, it has been shown that *Vibrio cholerae* toxin B subunit CT-B and its homologue *Escherichia coli* heat-labile enterotoxin, LT-B, can be used to target antigens to the immune responsive cells of the mucosa in the digestive tract where they are correctly processed (see e.g. Walker, 1994). The pentameric ring of B subunits binds specific receptors (primarily ganglioside GM1) on the mucosal gut epithelium and can enhance immunogenicity of other antigens when coupled herewith (see Jagusztyn-Krynicka et al., 1993). These findings have led to the development of recombinant enterotoxins as carrier molecules for the presentation of target protein antigens to the immune-responsive cells of the mucosal epithelium in mammals (Aitken and Hirst, 1993; Cardenas and Clements, 1993; Jagusztyn-Krynicka et al., 1993; Khoury and Meinersmann, 1995; Zhang et al., 1995). It has been shown that antigens fused to the carboxyterminus of CT-B or LT-B can elicit humoral and cellular immune responses to said target protein antigens in mammals and birds upon oral administration (Cardenas and Clements, 1993; Jagusztyn-Krynicka et al., 1993; Khoury and Meinersmann, 1995).

Studies on the immunological capacities of the GALT (gut associated lympoid tissue) of fish have confirmed the existence of a mucosal immune system also in fish (reviewed by Hart et al., 1988). Here, the intestine is also involved in the uptake of orally administered protein antigens (Dalmo et al., 1997; Lamers, 1985; Rombout and van den Berg, 1989; Rombout et al., 1985, 1989) and the production of mucosal immunoglobulins.

It has been shown that anal intubation of carp with either LT-B or LT-B fused to parvo peptide leads to uptake of these peptides in carp gut mucosa and the induction of anti-LT-B and anti-parvo peptide directed humoral immune responses (Companjen et al. Midtlyng PJ (ed): Fish Vaccinology. De v Biol. Basel, Karger, 2005, vol 121, pp 143-150). It will be understood that immunization of fish by anal intubation is not suitable for large scale application and that oral administration of antigens is obviously the preferred choice. The application of fusion of antigens to enterotoxin B-subunits as oral vaccine for fish as disclosed herein has not been reported before. CT-B subunits have been coupled to liposomes to enhance delivery of liposome-entrapped antigen (BSA) to the intestinal tract in fish (Irie et al., 2003). Liposomes without CT-B were also effective whereas no immune response was observed when fish were orally immunized with BSA-containing unstable liposomes or BSA alone. Thus, according to the teaching of Irie et al. it is of major importance for the induction of serum antibody responses in fish that antigens are protected by their encapsulation in stable liposomes. In marked contrast, the present invention now shows that it is not required to encapsulate or otherwise protect the antigen to induce a humoral immune response in fish following oral vaccination.

Provided herein is the production and use of an antigenic protein complex comprising an LT-B or CT-B subunit fused to an antigen of interest, wherein the protein complex is able to bind to a fish mucosal cell surface receptor, transported across the epithelium and exposed to the fish immune system to result in serum immune responses and protection.

In a preferred embodiment of the present invention, the protein complex comprises LT-B fused to an antigen of interest selected from the groups consisting of a viral, bacterial or microbial surface antigen of a fish pathogen. Examples of a virus or micro-organism pathogenic to fish include infectious pancreatic necrosis virus (IPNV), striped jack nervous necrosis virus (SJNNV), infectious haematopoietic necrosis virus (IHNV), viral haemorrhagic septicaemia virus (VHSV), Pancreas Disease virus (SPDV), infectious salmon anaemia virus (ISAV), Spring Viraemia of Carp virus (SVCV), Koi Herpesvirus (KHV), *Flexibacter columnaris, Edwardsialla ictaluri, E. tarda, Piscirickettsia salmonis, Vibrio* spp and *Aeromonas* spp., *Yersinia ruckeri, Pasturella piscicida* and *Renibacterium salmoninarum.* Of course, antigens that have been shown to be protective antigens in non-oral vaccine formulations in fish are of particular interest for the present invention. Known protective antigens that are suitably used in an oral vaccine formulation according to the invention include the G protein of Viral Haemorrhagic Septicemia virus (VHSV; Lorenzen et al., 1998).

Furthermore the invention provides a method for immunizing fish, comprising the oral administration of a composition comprising a protein complex of the invention. Preferably, oral administration of the protein complex comprises feeding fish with fish feed containing said protein complex.

A functional protein complex of the invention has a pentameric structure required for interaction with mucosal cell surface receptors. In one embodiment, it is a homopentamer of five identical antigen-B-subunit fusion proteins. One LT-B subunit can be fused to a single antigen or to multiple copies of that antigen. It is also possible to incorporate more than one type of antigen in an LT-B or CT-B-based protein complex. For example, a tandem repeat of different types antigens can be fused to one B-subunit. In an alternative embodiment, the antigenic protein complex is a heteropentameric complex composed of five B-subunits fused to different antigens. For example, two subunits of the complex are fused to antigen A and three subunits of the complex are fused to antigen B. Other combinations are of course also possible. Furthermore, not all B-subunits need to be fused to an antigen of interest. Use of protein complexes consisting of at least one 'free' or "unaltered" B-subunit and at least one fused B-subunit as an oral fish vaccine is also encompassed. In fact, it is believed that for certain antigens, in particular large antigens, it is preferred that not all subunits of the pentamer are loaded with antigen because the antigen may otherwise interfere with the formation of a functional pentamer (see application PCT/NL2004/000708).

An LT-B or CT-B-based protein complex comprising an antigen of interest can be prepared using a host cell provided with the suitable nucleic acid construct(s) encoding the components of the complex and allowing expression and assembly of said components into a functional complex. Host cells that can be used for the production of a protein complex include plant cells, fish cells, yeast cells (e.g. *Pichia pastoris*), algae, for example brown algae such as *Egregia enziesii* or green algae such as *Chlamydomonas rheinhardtii,* mammalian cells, fungal cells and insect cells. Suitable fungal cells include *Agaricus bisporis, Cantharellus cibarius, Pleurotus* spp. and *Coprinus* spp. Bacterial host cells may be used, for example commensal lactic acid bacteria such as *Lactococcus lactis* or *Lactococcus plantarum.* Preferably, a host cell is an edible host cell which does not cause harm upon consumption. In a specific embodiment, a plant can be used to produce an immunogenic protein complex of the present invention. For example, plant cells belonging to monocots or dicots such as corn or rice or potato or tobacco can be used.

Standard recombinant DNA technology can be used to prepare the required constructs. The construct can be introduced in the host cell by various conventional techniques, including transfection, electroporation and *Agrobacterium-*mediated gene transfer. A person skilled in the art will be able to choose the most suitable technique for a particular host cell.

Surprisingly, it was observed for some antigens of interest that the coupling of antigen to a B-subunit enhances the expression of the antigenic protein when expressed in a host cell. In particular, a nucleic acid sequence encoding a fusion of LT-B and the viral glycoprotein (G) of the fish viruses VHSV or SVCV allowed for optimal transcription and translation initiation in plant host cells. In contrast, the nucleic acid sequence encoding only the viral antigen was not or only very poorly expressed in the host cell. Plant host cells used as recombinant expression system are typically reared in a glasshouse at a temperature between 18 and 30°C. The optimal temperature for viruses that are pathogenic for cold water fish (e.g. VHSV and SVCV) is much lower; it has been observed that the fish viruses VHSV and SVCV flourish best between 8 and 14°C. Furthermore, no pathogenicity of these viruses is observed at higher temperatures. This may indicate that recombinant expression of the viral G proteins alone in a plant host cell is hampered by the fact that the plants are grown at a temperature (18-30°) well above the temperature at which the antigens are normally expressed (8-14°C). Both viral glycoproteins are multimeric proteins and it is conceivable that their folding and multimerization cannot take place properly at supra-optimal temperatures such that they are more susceptible for proteolytic degradation. Without wishing to be bound by theory, it is proposed that the coupling of the viral antigens to LT-B (or CT-B) subunits that are capable of forming pentameric complexes when expressed in plants enhances the stability and expression of the antigen. The invention therefore also relates to a method for the expression of a protein of interest in a (plant) host cell wherein said protein originates from an organism that has an optimal growth temperature that lies below the optimal growth temperature of said host cell, wherein said protein is expressed as a fusion protein with a B-subunit of *Vibrio cholerae* cholera toxin (CT) or *Escherichia coli* heat-labile enterotoxin (LT). Said host cell is for example a plant cell and said protein of interest is for example derived from a marine animal or an organism pathogenic to a marine animal. The method is advantageously used for the expression of a protein of interest that in its native form exists as a multimer, for example as a dimer or a trimer. Following expression in the (plant) host cell, the fusion protein can be used as such (e.g. as an antigenic protein complex of the invention in an oral vaccine composition). Alternatively, the fusion protein can be further processed to remove the B-subunit and release the protein of interest. Processing can be performed enzymatically, e.g. using a protease like trypsin, or chemically.

In a further aspect, a protein complex comprising an antigen of interest as disclosed herein is advantageously formulated into an oral vaccine composition that can be administered to fish in a non-labour-intensive manner, e.g. being part of fish feed particles or pellets, and causes no stress to the fish, in complete contrast to conventional vaccine administration by parenteral injection. Herewith, the invention provides a fish feed composition comprising a protein complex comprising an antigen of interest fused to the B-subunit of *Vibrio cholerae* cholera toxin (CT-B), or to the B-subunit of *Escherichia coli* heat-labile enterotoxin (LT-B). A fish feed composition of the invention for instance comprises feed pellets or particles to which a protein complex of the invention has been added either during or after manufacture of the fish feed. Dry pellet fish feed is getting popular among aquaculture industry in the last few years. Pelleted commercial fish feeds are available in a variety of pellet sizes. The main ingredient of most types of dry pellet fish feed is fish meal, protein from other animal or plant origin, fish oil or other kinds of lipids, vitamin premix, minerals and binders in accordance with the nutritional requirement of the target cultured species. The combined ingredients are usually extruded into pellets of different sizes and densities to suit the feeding behaviour of different types of cultured fish. The choice of the size of pellet to feed is typically based on the size of the fish. In one embodiment, fish pellets prepared in the conventional manner are coated with a composition comprising an antigenic protein complex of the invention. Preferably however, pellets are prepared from a mixture of the conventional pellet ingredients and a protein complex of the invention. The protein complex can be added to fish feed in a crude or a (partially) purified form. For example, if an edible host cell is used for the production of a protein complex, the host cells expressing the components of the complex can be used as such in fish feed. In a specific embodiment, feed pellets are provided that contain a certain amount (e.g. 20% by weight) of freeze-dried potato tuber material obtained from transgenic potato host cells expressing LT-B ― antigen fusion protein. This corresponded to approximately 4-5 µg fusion protein per gram of food. Feeding these pellets to fish resulted in a systemic humoral immune response to the antigen.

**The invention is illustrated by the Examples below.**

### LEGENDS

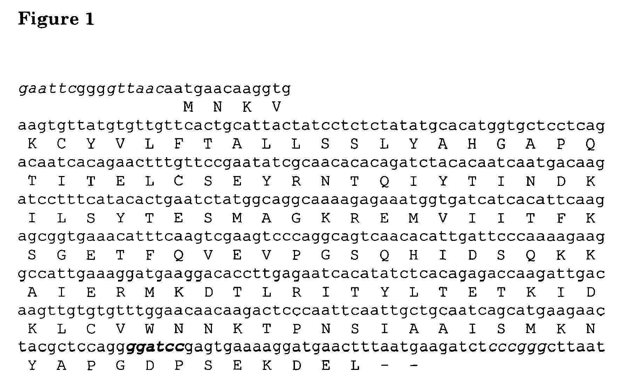

Figure 1. Gene sequence synthetic gene for LT-B as present in pLANTIGEN4. In italics are *Eco*RI, *Hpa*I, *Bam*HI and *Sma*I sites used for cloning. Putative translation is given in single letter amino acid abbreviation.

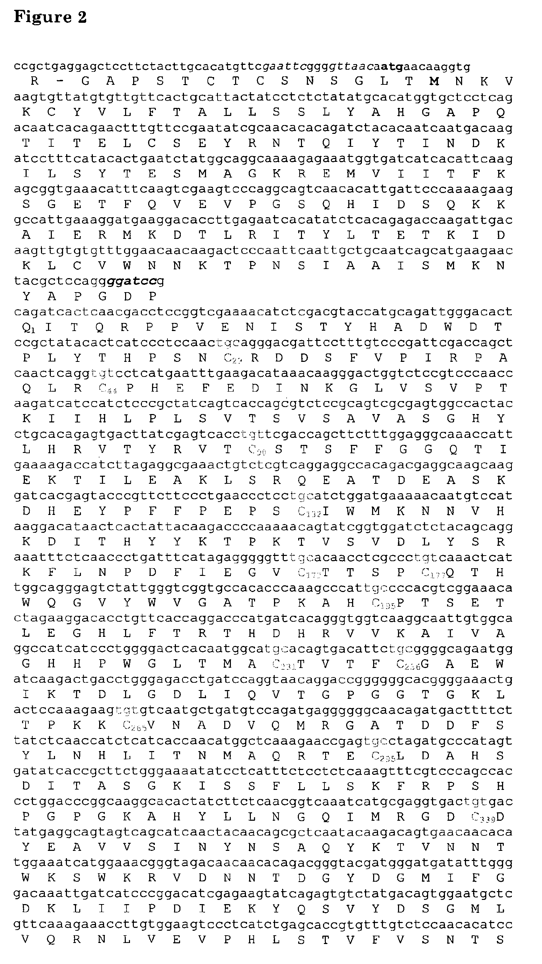

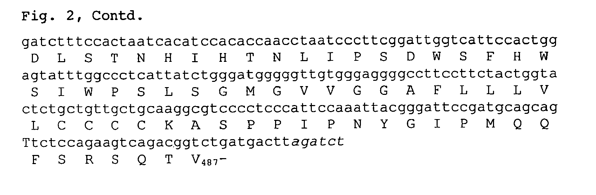

Figure 2. Gene sequence fusion synthetic gene for LT-B and VHSV G as present in pLANTIGEN24. Putative translation is given in single letter amino acid abbreviation.

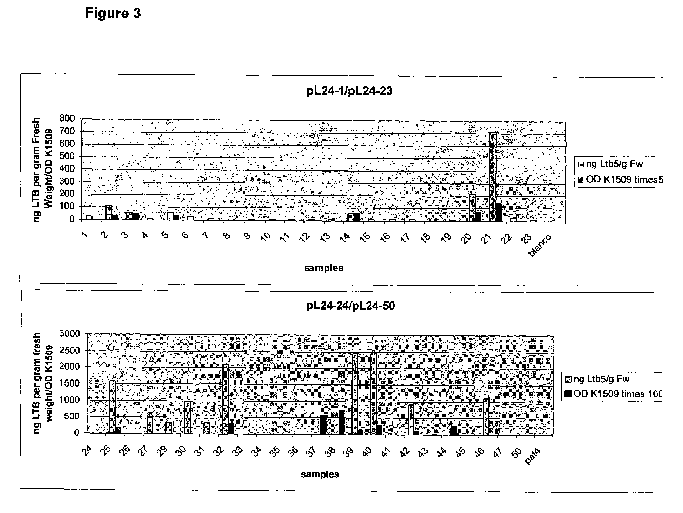

Figure 3. Results of GM1 ELISA (light bars) and modified GM1 ELISA using K1509 polyclonal antibody against VHSV G (dark bars), for all pLANTIGEN24 transgenic tuber plant extracts. Panel A shows the results for plants 1 to 23. Panel B shows the results for plants 24 to 50. Five micrograms of total potato tuber extract was loaded onto GM1 plate. Detection was with VD12 (Lauterslager et al., 2001) for the presence of LT-B5 pentamers (Ltb5) and K1509 for the presence of VHSV G.

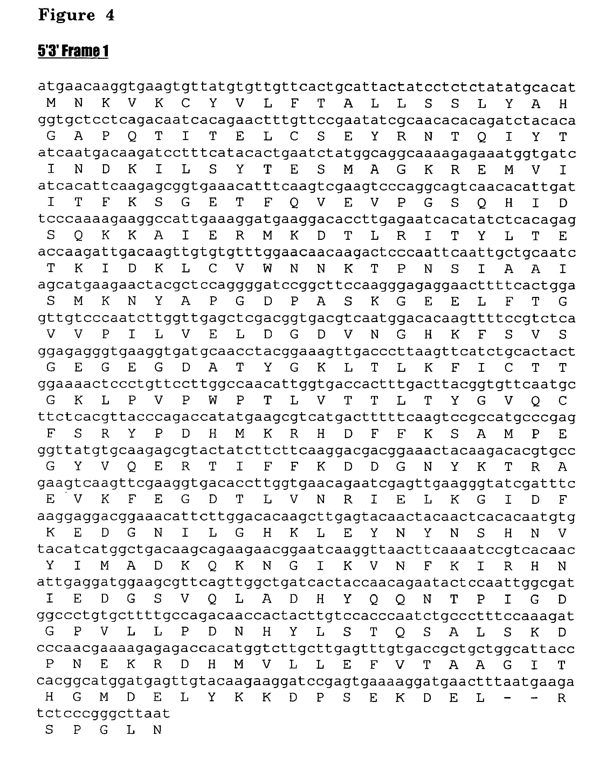

Figure 4. Gene sequence fusion synthetic gene for LT-B and GFP sequence as present in pLANTIGEN20. Putative translation is given in single letter amino acid abbreviation.

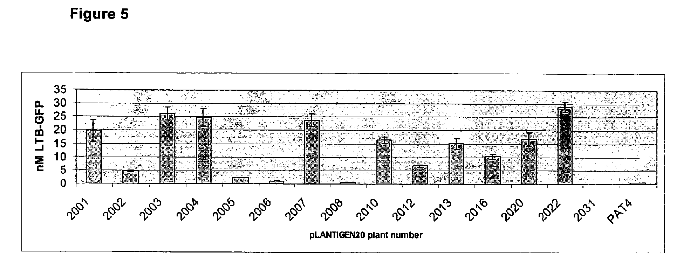

Figure 5. Results of GM1 ELISA for selected pLANTIGEN20 (LT-B-GFP) transgenic tuber plant extracts. Detection was with VD12 (Lauterslager et al., 2001) for the presence of LT-B5 pentamers. PAT4, negative control. Concentration is given in nM.

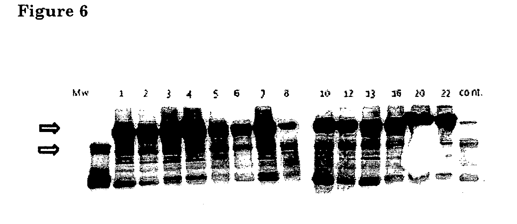

Figure 6. Western blot analysis of pLANTIGEN20 (LT-B-GFP) transgenic tuber extracts under semi-native conditions. Numbers above the lanes indicate the individual plant number (lanes 3 to 16). Lane 2, pLANTIGEN4 (LT-B) positive control. Cont. Refers to extract of a PAT4 (empty vector) negative control. Blots were probed with the monoclonal antibody VD12 to detect pentameric complexes of LT-B. The upper arrow indicates the position of a high molecular weight complex of the LT-B-GFP complex whereas the lower arrow indicates the LT-B complex of appr. 60 kDa.

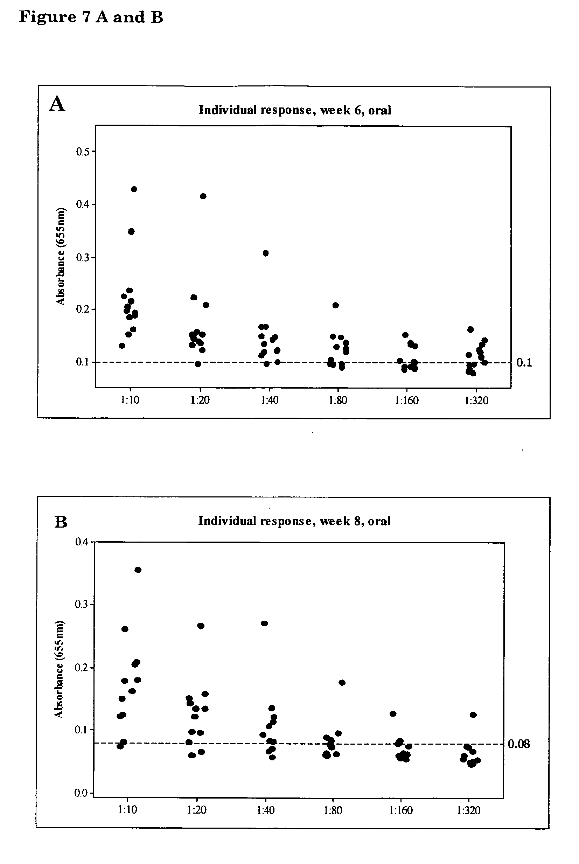

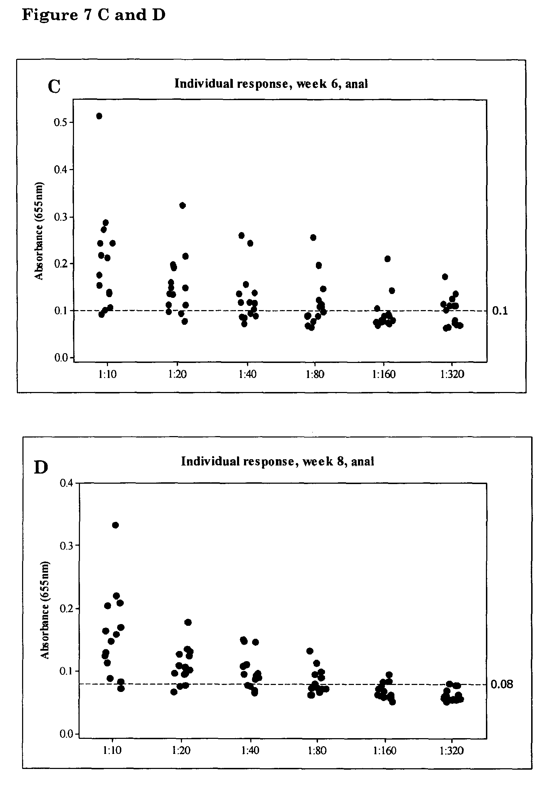

Figure 7. Results of oral (panels A en B) and anal (panels C and D) immunization experiments of trout with LT-B-GFP protein complex produced in potato tubers. Six (panels A and C) and eight weeks (panels B and D) after immunization sera was collected. Various dilution of the sera (1:10 to 1:320) were assayed for the presence of anti-GFP antibodies by an Elisa assay using GFP-coated plates, mouse anti-trout and horseradish-peroxidase coupled goat anti-mouse antibodies. The absorbance at 655 nm is reflective of the amount of complex formed between GFP and anti-GFP antibodies in the serum (for details see Example 3). Test sera are considered positive if the absorbance was larger than two-fold the negative control sera absorbance (indicated by dotted lines).

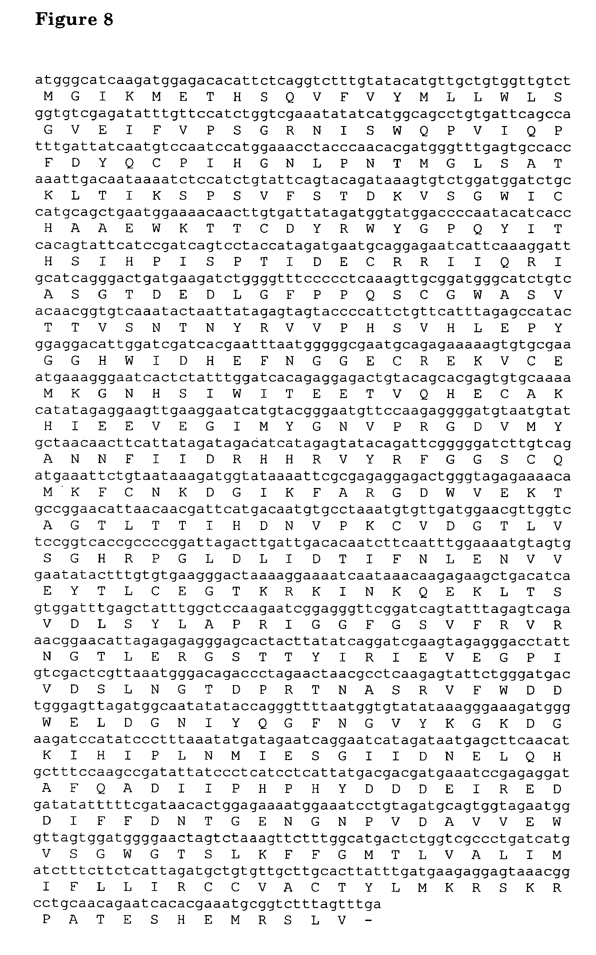

Figure 8. Gene sequence of SVCV-G as present in pLANTIGEN25. Putative translation is given in single letter amino acid abbreviation.

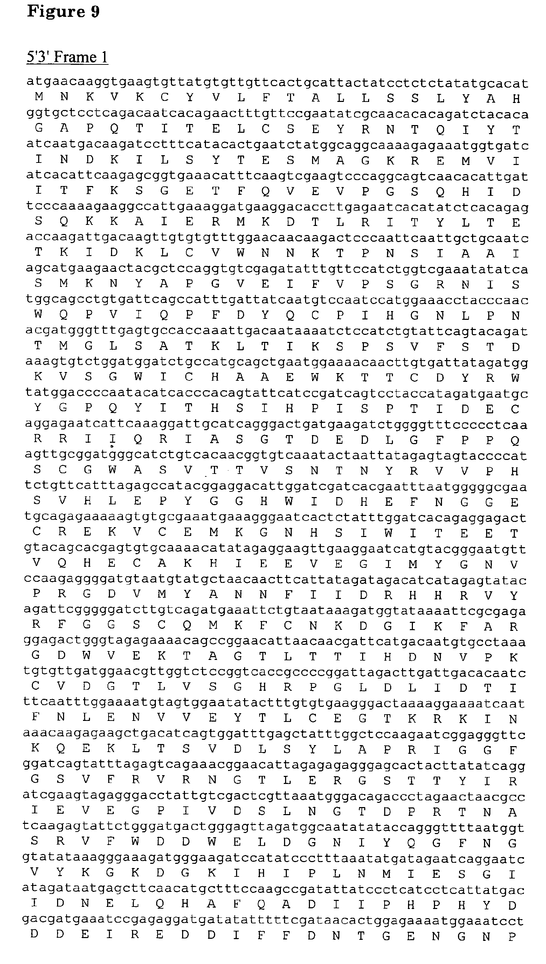

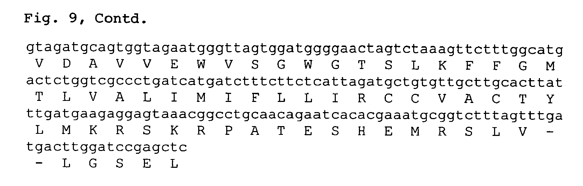

Figure 9. Gene sequence fusion synthetic gene for LT-B and sequence SVCV-G as present in the fusion construct pLANTIGEN27. Putative translation is given in single letter amino acid abbreviation.

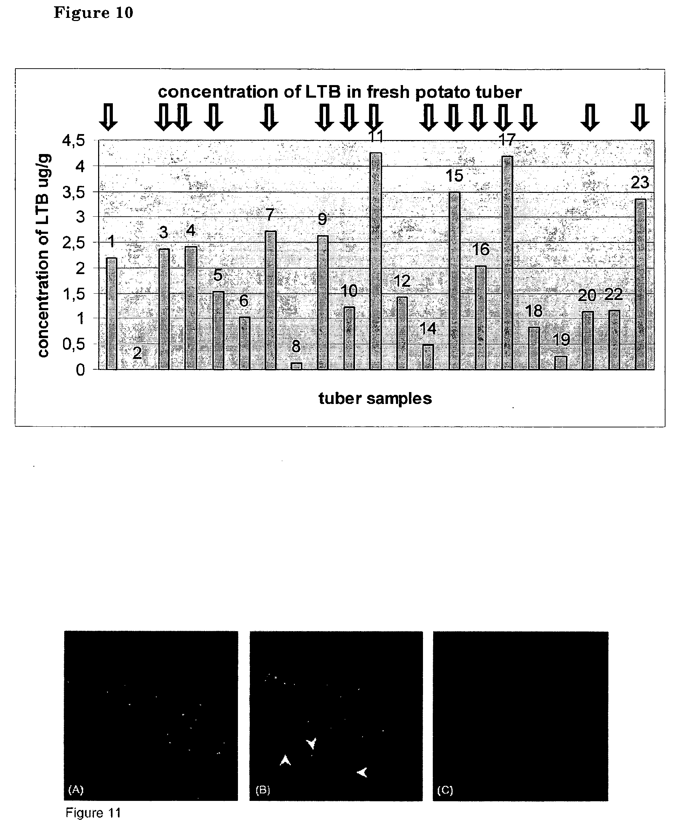

Figure 10. Analysis of functional LT-B pentamers in tuber extracts of transgenic plants transformed with a mixture of pL4 (encoding LT-B) and pL27 (encoding the LT-B-SVCV-G fusion protein). Pentamer formation was determined by GM1 ELISA. To analyse the presence of SVCV G protein in the pentameric complexes, a modified GM 1 ELISA was performed in which the detection of bound complexes with VD12 (specific for LT-B5) was now done with monoclonal antibody 2C1/3C9 specific for SVCV G. Tuber samples that reacted positively in this modified GM1 ELISA are indicated in with an arrow.
Figure 11. LTB facilitates a better uptake of GFP in carp gut mucosa. Sections of carp gut intubated with (panel A) GFP, (panel B) LTB-GFP containing potato suspension and (panel C) control potato suspension and incubated for 6 hours. Note the large macrophage-like cells in the LTB-GFP intubated gut (arrowheads).

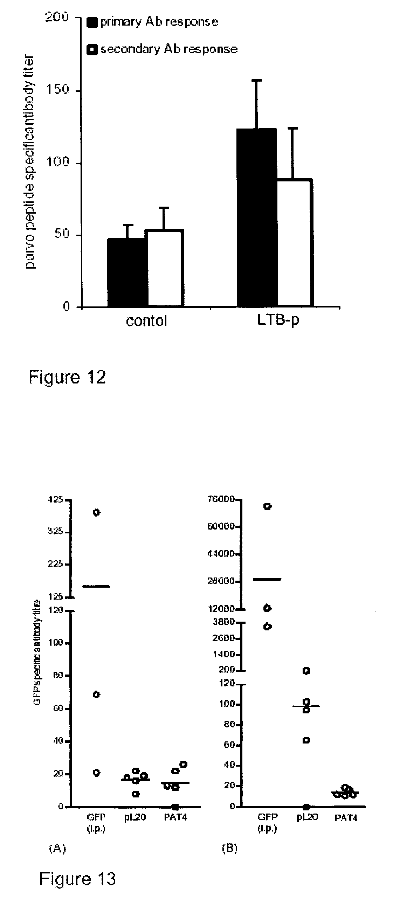

Figure 12. Systemic parvo peptide - directed humoral immune responses are induced upon anal intubation. Parvo peptide-specific antibody responses were measured in primary and secondary immune serum of fish anally immunised with LTB-p (LTB-parvo peptide). The parvo peptide specific antibody responses were measured by ELISA. The mean antibody titre ± SEM of 3 fish is shown.
Figure 13. Oral immunisation with pL20 (LTB-GFP) treated food pellets results in an anti-GFP response. Carp were orally immunised by feeding with pL20 or PAT4 (empty vector control) mixed food pellets or pL20 or PAT4 coated food pellets. Anti-GFP responses in (A) primary immune serum and (B) secondary immune serum are shown. Anti-GFP responses were measured by ELISA. GFP i.p.: anti-GFP responses in serum of fish i.p. immunised with GFP in IFA.

### EXAMPLE 1. Oral vaccine for Infectious Pancreatic Necrosis

*Introduction.* Infectious Pancreatic Necrosis (IPN) is a viral disease caused by IPN virus (IPNV), the prototype virus of the family *Birnaviridae*. It is an important viral disease that affects both salmonid and non-salmonid finfish species and is distributed worldwide. Only specific data for Norwegian Atlantic salmon industry are available where estimated losses due to IPN are estimated to be approximately 60 million ECU annually (Christie, 1997). The world-wide enzootic distribution of IPNV, and other birnaviruses, suggests that their successful control will be crucial for future cultivation of both new and existing fish species. IPNV possesses a double-stranded bi-segmented RNA genome. Its viral particles consist of an unenveloped icosahedral 60 nm capsid. The viral genome is transcribed into two non-polyadenylated sequences corresponding in size to the A and B segments of the genome. The sequence of the viral A segment encodes an approximately 100 kDa polyprotein which is cleaved to produce, in order from the amino terminal end, the major virion protein VP2, and the minor structural proteins VP4 and VP3. The major capsid protein VP2 is produced from a precursor protein pVP2 and is then assembled to form the capsid. VP2 protein was shown to be protective and is the sole antigen present in a commercially available injection vaccine.
***Gene construct**.* A genetic fusion of a synthetic gene for LT-B, optimized for expression in potato and other Solanaceae, and the coding sequence for the VP2 major capsid protein of IPNV can be made as follows: the gene coding for the major capsid protein VP2 of IPNV is adapted for cloning into the unique BamHI site of pLANTIGEN4 in frame at the C terminus of the synthetic gene for LT-B (Lauterslager et al., 2001; Figure 1) by PCR amplification of a template resembling the gene sequence of genbank accession U48225 using primers IPNVfor 5'-caggggatcccatgaacacaaacaaggcaaccgc-3' and IPNVrev 5'-aaaaacccgggagatctcattacacctcagcgttgtctccgc-3'. The respective BamHI/SmaI fragment can be cloned under control of the patatin promoter in pLANTIGEN4 (Lauterslager et al., 2001) to generate pLANTIGEN32 (LT-B-IPNV VP2). Transgenic plants can be made as described in Lauterslager et al. (2001) and plants can be selected by GM1 ELISA of potato tuber extracts.

### EXAMPLE 2. Oral vaccine for VHSV

***Gene constructs and transformation***. To enable recombinant VHSV G protein synthesis in transgenic plants, gene construct pLANTIGEN21 was made and transformed into potato. PLANTIGEN21 was made as folows: unique SalI and BglII sites were introduced at the N- and C-terminus of the mature G protein coding sequence of VHSV G by amplification of pcDNA3vhsG (McLauchlan et al., 2003) with oligonucleotides VHSV G 1, 5'-tctggtgtcgaccagatcactcaacgacctccgg-3' and VHSV G3, 5'-gatcgaagatctaagtcatcagaccgtctgacttctg-3' by PCR under optimum conditions using a proof-reading Pwo polymerase for amplification. An NcoI/SalI fragment comprising a signal peptide for secretion (Van Engelen et al., 1994) was ligated to the respective SalI/BglII digested PCR fragment and placed under control of the patatin class I promoter and nopaline synthase terminator by cloning the resulting NcoI/BglII fragment in the NcoI/BamHI sites of pLANTIGEN4 (Lauterslager et al., 2001; Figure 1) to generate pLANTIGEN21. The latter was transformed to potato as described (Lauterslager et al., 2001) and 58 transgenic plants were regenerated and grown to maturity in the greenhouse.
Accordingly, a genetic fusion of LT-B and the G protein of VHSV was constructed by introducing an unique BamHI site at the N terminus of VHSV G protein coding sequence in pLANTIGEN21, and an unique SmaI site at the C-terminus, by PCR amplification using primers G-F, 5'-caggggatcccagatcac-3' and Glong-R-SmaI, 5'-aaaaacccgggagatctcattaaagttc-3'. The resulting BamHI/SmaI fragment was cloned in frame in the unique BamHI site at the C-terminus of LT-B coding sequence in pLANTIGEN4 (Lauterslager et al., 2001) generating pLANTIGEN24 (Figure 2). The latter was transformed into potato as described and 47 independent transgenic plants were regenerated and grown to maturity in the greenhouse.
***Analysis of expression**.* Tuber extracts of pLANTIGEN22 tubers were analysed for the presence of VHSV G protein by sandwich ELISA using monoclonal antibodies 3F1A2, IP1H3 and 3F1H10 (Cupit et al., 2001) as described (Lorenzen et al., 2000). None of the extracts of transgenic plants showed expression of the G protein. Potato tuber extracts of all pLANTIGEN24 tubers were analysed by GM1-ELISA for the presence of LT-B5 pentamers (Lauterslager et al., 2001). To enable detection of VHSV G protein, a modified GM1-ELISA was performed by incubating microplates coated with purified bovine brain GM1 ganglioside with potato tuber extracts, followed by incubation with K1509 a polyclonal antibody against VHSV G recognizing viral VHSV G protein. Using this assay, the presence of VHSV G protein in a complex with pentameric LT-B5 can be established. The results of both GM1 ELISA and the latter modified GM1 ELISA are summarized in Figure 3 for all pLANTIGEN24 plants. As can be seen from Figure 3, by making a fusion protein comprising LT-B and the VHSV G protein, GM1 binding LT-B-VHSV G protein complexes can be established in transgenic potato tubers. There is a strong correlation between the level of GM1 binding activity and recognition by the polyclonal K1509 antibody of GM1 bound complexes, as expected. At least 19 transgenic plants showed expression of GM1 binding LT-B above background (approximately 1 nanogram/gram fresh weight).
More than half of these also were positive for VHSV G protein whereas none of the pLANTIGEN22 was. Levels were up to 2.5 micrograms of LT-B5 per gram fresh weight tuber. Selected plants pL2420 (i.e. plant number 20 of the group of plants transgenic for pL24) and pL2421 were also positive in a sandwich ELISA with the two monoclonal antibodies 3F1A2 and IP1H3, suggesting proper folding of at least the two conformational epitopes that are recognized by these two mAbs (Lorenzen et al., 2000).
***Immunogenicity and challenge experiments**.* Extracts and freeze dried tuber material of selected plants including pL2421, are used in a vaccination trial and challenge experiment. Rainbow trout fingerlings of approximately 4 grams (120 per group) are immunized intraperitoneally by injection of 50 microliters of a 10% pL2421 extract mixed with Freunds incomplete adjuvant, intramuscularly by injection of 25 microliters of a 10% homogenate in phosphate-buffered saline (PBS) or orally by application of 100 microliter of 5% homogenate in fish oil, twice and challenged 6 weeks post vaccination with VHSV. Sera are collected 6 weeks post vaccination to study antibody responses. Challenges are as described (McLauchlan et al., 2003). Alternatively, freeze dried tuber material of selected transgenic plants expression LT-B complexes and positive for VHSV G, including pL2420, pL2421, pL2439 and pL2440, are incorporated into a standard fish meal and resulting pellets are used for oral in feed immunization.

### EXAMPLE 3. Oral and anal intubation of trout with LT-B-GFP

***Gene construct LT-B-GFP.*** A gene fusion of LT-B and green fluorescent protein (GFP) was made as follows: a unique BamHI site was introduced in the coding sequence of a GFP sequence by PCR using oligonucleotides GFPFw 5'-aggggatccggcttccaagggagaggaac-3' and GFPRev 5'-ctcggatccttcttgtacaactcatccatgcc-3'. The resulting BamHI fragment was cloned in the unique BamHI site at the C-terminus of a synthetic gene for LT-B in pLANTIGEN4 (Lauterslager et al., 2001; Figure 1) to generate a translational fusion LT-B-GFP named pLANTIGEN20 (Figure 4). The latter binary vector was introduced in *Agrobacterium tumefaciens* strain Ag10 and used for transformation of potato cultivar Desiree as described before (Lauterslager et al., 2001).

***Tuber analysis*.** Thirty-one (31) transgenic plants were generated and grown to maturity in the greenhouse. Tuber tissue slice analysis for green fluorescence at 480 nm indicated that almost all were positive for GFP. Half of the transgenic tubers were analysed by GM1-ELISA for the presence of LT-B5 pentameric complexes and results are summarized in Figure 5. Western blotting under semi-native conditions and using the LT-B5 conformational monoclonal antibody VD 12 for detection of pentameric complexes, indicated the presence of high molecular weight complexes in transgenic plants positive in GM1 ELISA (Figure 6). From Figure 6 it can be seen that all transgenic pL20 plants accumulated high molecular weight complexes that are recognized by VD12 (left upper arrow) whereas for control pL4 plant expressing the synthetic gene for LT-B, an approximately 60 kDa complex can be visualized (lower arrow). Plant pL2003 was selected which showed expression of LT-B-GFP pentameric complexes at approximately 25 nM scale which is equivalent to 5.3 micrograms of complex per gram fresh weight (Figure 5). pL2003 was further grown in the greenhouse for bulk tuber production. Tubers were harvested, peeled and freeze-dried. Fish were either immunized with freeze-dried pL2003 material or with fish feed comprising 20% of pL2003 freeze-dried tuber material.

***Immunization experiments rainbow trout**.* Rainbow trout (mean weight 84.9g) were immunised by oral or anal intubation with selected freeze-dried pL2003 homogenized potato tuber material comprising approximately 25 micrograms of LT-B-GFP per gram dry weight. Prior to immunisation, fish were starved for 24h. Potato tuber material expressing LT-B-GFP was passed through a 100 µm mesh utilising a pestle and then suspended in PBS (0. 15M, pH 7.2) to a final concentration of 200 mg DW/ml which approximates 5 micrograms/ml of LT-B-GFP. Trout were anaesthetised by exposure to benzocaine (50mg/l) and were either orally, 200 microliter (effective concentration 1 microgram LT-B-GFP) or anally immunised with 100 µl of resuspended tuber material (effective concentration 500 nanograms LT-B-GFP) via a short section of plastic tubing attached to a 1ml syringe. Groups consisted of 15 fish per delivery route and fish were identified by marking them sub-cutaneously with alcian blue dye. Fish were then returned to holding tanks, 1m in diameter, 3401 in volume 151/min. Temperature for the experimental period ranged from 8.7 to 14.2°C. Fish were bled at weeks 6 and 8 post-intubation. Blood was collected, allowed to clot overnight at 4°C and then centrifuged at 3500rpm for 15 mins and the sera collected, aliquoted and frozen at -80°C until assayed. Purified recombinant GFP expressed in E. coli and purified was used for coating. Ninety-six well ELISA plates (Immulon 4, Dynex) were coated with *E. coli* expressed GFP (50µl 10µg/ml in carbonate-bicarbonate buffer 0.05 M, pH 9.6) and left overnight at 4°C. Plates were washed with PBS containing 0.05% Tween 20 (PBST, 3x2min) and blocked with 5% dried non-fat skimmed milk in PBST (100µl/well) for 2h at 37°C. Plates were washed as before and frozen at -20°C. Sera were tested as follows. Ninety microlitres of PBS containing 1% bovine serum albumin was added to each well. Ten microlitres of test fish sera was added to column 1 and 7 and doubly diluted across the plate to columns 6 and 12 respectively, giving a dilution range of 1: 10 to 1:320. Sera were incubated for 2h at room temperature before washing as before. Subsequently, 50µl I-14 (monoclonal to trout Ig, 1:2 PBST) was added and incubated for 1h at 37°C. Plates were washed as before, and 50µl goat anti-mouse horseradish peroxidase (HRP) conjugate (Sigma) was added (1:70,000 PBST) and incubated for 1h at 37°C. Plates were then washed 2x2min PBST followed by 1x2min PBS and 100µl tetramethylbenzidine (TMB) substrate (Sigma) was added and plates incubated for 30 min in dark. Plates were then read at 655nm and the absorbance recorded. Each plate consisted of test sera, known positive sera (from fish i.p. injected with GFP in Freunds complete adjuvant) and negative sera. In all plates, positive sera exhibited absorbencies that were approximately 10 fold higher than the negative sera. Test sera were considered positive at each dilution if the absorbance was greater than 2x the negative control sera absorbance. The quoted titre is therefore the last one that was 2x that of the negative control. Results are summarized in Figure 7. Figure 7 shows that 6 weeks post oral immunization (panel A) all fish have immune resonses above background at 1:10 dilution and more than half of the fish also at 1:320 dilution. Antibody responses persist at 8 weeks post oral immunization (panel B) whereas antibody responses upon anal intubation (panels C and D) slightly decrease in time (8 weeks, panel D compared to 6 weeks post immunization, panel C).

### EXAMPLE 4. Oral vaccine for SVC in carp

**Gene constructs and transformation.** To enable recombinant SVCV G protein synthesis in transgenic plants, the gene construct pLANTIGEN25 was made and transformed into potato. PLANTIGEN25 was made as follows: unique XhoI and BamHI sites were introduced at the N- and C-terminus of the mature G protein coding sequence of SVCV G by amplification of pcDNA3-svcG-539 with oligonucleotides SVCVG1, 5'-tctggtctcgagatccccatatttgttccatc-3' and SVCVG2, 5'-gatcgaggatccaagtcatcaaactaaagaccgcatttcg-3' by PCR under optimum conditions using a proof-reading Pfu polymerase for amplification. An NcoI/XhoI fragment comprising a signal peptide for secretion (Van Engelen et al., 1994) was ligated to the respective XhoI/BamHI digested PCR fragment and placed under control of the patatin class I promoter and nopaline synthase terminator by cloning the resulting NcoI/BamHI fragment in the NcoIBamHI sites of pLANTIGEN4 (Lauterslager et al., 2001; Figure 1) to generate pLANTIGEN25 (Figure 8). The latter was transformed to potato as described (Lauterslager et al., 2001) and 47 transgenic plants were regenerated and grown to maturity in the greenhouse. In addition, a genetic fusion of LT-B and the G protein of SVCV was constructed as follows: unique NcoI and SalI sites were introduced in the synthetic gene for LT-B by amplification of pLANTIGEN4 with oligonucleotides LTBsal, 5'-gagtcgtcgacacctggagcgtagttcttcatgc-3' and LTBnco, 5'-gtgacgaagacaacatgaacaaggtgaagtgttatgt-3' using a proof reading Pfu polymerase under optimum conditions. BpiI/SalI digested PCR fragment comprising the synthetic gene for LT-B, was cloned in NcoI/SalI digested binary vector pLANTIGEN25 and the resulting plasmid was cut with SalI and BamHI. A XhoI/BamHI fragment of pLANTIGEN25 comprising the sequence for SVCV G was ligated in the former digested plasmid resulting in pLANTIGEN27 comprising an LT-B-SVCV G fusion (Figure 9). *Solanum tuberosum* Desiree was transformed with a 1:1 mix of pLANTIGEN4 and pLANTIGEN27 (co-transformation) using *Agrobacterium tumefaciens* -mediated transformation. 21 independent transgenic plants were generated for pLANTIGEN(4+27) and grown to maturity in the greenhouse.

**Analysis of expression**. Tuber extracts of pLANTIGEN25 tubers were analysed for the presence of SVCV G protein by standard ELISA or sandwich ELISA using various combinations of monoclonal antibodies G3C7, 4C12, 2C1/3H1, 2C1/3G2, 2C1/3C9, 2C1/A10/2G2, 2C1/A10/1H11 and 2C1/A10/1D12 all raised against SVCV CZ539 strain and specific for G protein. None of the 47 plants exhibited expression of SVCV G. Analysis of pL(4+27) tuber extracts was initially performed by GM1 ELISA. Results are summarized in Figure 10 and indicate that a number of transgenic plants show expression of GM1 Binding LT-B5 pentamers up to 4 micrograms/g FW tuber. To analyse the presence of SVCV G protein, a modified GM1 ELISA was performed in which the detection of bound complexes with VD12 (specific for LT-B5) was now done with monoclonal antibody 2C1/3C9 specific for SVCV G. Extracts from pL(4+27) plants that reacted positively in this modified GM1 ELISA are indicated in Figure 10 with an arrow. As can be seen, most of the plants that were positive for GM1 binding LT-B5 also reacted with 2C1/3C9 showing the presence of SVCV G protein in the complex.

**Immunogenicity and challenge experiments**. Extracts and freeze dried tuber material of selected plants including pL(4+27)-11, 15, 17 and 23 will be used in a binding and uptake experiment with carp and vaccination trial and challenge experiment. A mix of freeze-dried tuber material comprising pL(4+27)-11, 15, 17 and 23 was incorporated into a standard fish feed to final concentration 20% tuber material as described in EXAMPLE 5. The resulting feed will be used for oral immunization of carp as described. Carps reared at 10°C will be challenged 1, 12 and 21 weeks post vaccination by intraperitoneal injection of 0.2 ml of SVCV virus strain 539.

### EXAMPLE 5. Oral immunization of carp

**Gene constructs and transformation.** Design and construction of the pLANTIGEN4 synthetic plant-optimized gene for expression of LTB in potato tuber and control PAT4, have been described previously (Lauterslager at al., 2001). pLANTIGEN15 was constructed by cloning two synthetic influenza virus hemagglutinin (HA) heavy chain decapeptide sequences, representing amino acids 111-120 of PR8 HA-1 (Hackett et al., 1985), together with two synthetic sequences coding for the amino-terminal region of the viral VP2 protein of canine parvovirus (CPV; Langeveld et al., 1994) into the unique BamHI site of pLANTIGEN4. Each of the four sequences was cloned in such a way that they were separated by two alanine residues for spacing (LT-B-iipp). pLANTIGEN20 was constructed by cloning a sequence for green fluorescent protein (GFP) in frame into the unique BamHI site of pLANTIGEN4 as described in Example 3. Transformation of *Solanum tuberosum* cultivar Désirée, growth of transgenic plants and tuberisation are as described (Lauterslager et al., 2001).

**Tuber protein and fish feed preparations.** One transgenic line for pLANTIGEN 15 (pL1516) was selected based on a combination of good tuber setting and recLT-B production as estimated by GM1-ELISA. Identification and analysis of pL2003 harboring an LT-B-GFP fusion protein was described in Example 3. PAT4 control line comprising an empty vector cassette was described before (Lauterslager et al., 2001). All, pL1516, PAT4 and pL2003, were multiplied through seed tubers and grown in the greenhouse for bulk tuber production. Bulk tubers were harvested, peeled, cut into slices, freeze dried and homogenised by grinding using a mortar and pestle. Ground and homogenized material was used in immunization experiments with carp and for manufacturing a fish feed for oral immunizations. Incorporation into fish feed pellets was done by mixing 20% (final concentration) of pL2003 homogenized freeze-dried potato tuber material (approximately 21 micrograms of LT-B-GFP per gram dry weight tuber) with normal fish feed compounds prior to making the pellets. The resulting mix containing the potato material was converted into pellets and coated with fish oil according to standard methods. The feed was dried at room temperature before use. Final concentration of LT-B-GFP in feed was estimated 4.3 micrograms/g feed.

**Carp binding and uptake experiments**. Six month old carp *(Cyprinus carpio L.)* weighing around 20 g, were reared in re-circulating, filtered and UV-sterilised water at 3°C. The fish were fed with standard food pellets (Skretting/Nutreco, Putten, The Netherlands) at a daily ration of 2.5 % of their body weight. Twenty-four hours prior to an intubation or in feed immunization experiment, the fish were fasted. pL2003 homogenized freeze-dried tuber material or control PAT4 were resuspended in PBS to approximately 0.4 µg LT-B-GFP/100 µl suspension (pL2003 suspension). recGFP purified from *E. coli* extracts was diluted in PBS to final concentration 13.5 µg/100 µl. Groups of 3 fish were anally intubated with 100 µl recGFP suspension (13.5 µg), pL2003 suspension (approximately 0.4 µg LT-B-GFP) or PAT4. Six hours post intubation the fish were killed by an overdose of Tricaine Methanesulfonate (TMS, Crescent Research Chemicals, AZ) and the end gut was removed, snap froozen in liquid nitrogen and stored at -80°C prior to analysis. Gut tissue was cut using a cryostat (Reichert-jung 2800 Frigocut N, Nussloch, Germany) and tissue sections were embedded in vectashield (Vector Laboratories Inc., Burlingame, CA), containing propidium iodide. Gut section analysis by laser scan microscopy (Zeiss LSM-510, Jena, Germany) clearly showed that LT-B-GFP (pL2003 material) is taken up more efficiently compared to recGFP (Figure 11). GFP could be detected in the supranuclear vacuoles of enterocytes and also in macrophage-like cells underlying the epithelium suggesting transport from enterocytes into large macrophage-like cells (Figure 11B). Although little GFP could be detected in enterocytes of fish intubated with GFP only, the signal was less intense compared to the LT-B-GFP treated fish and virtually no GFP could be detected in the macrophage-like cells (Figure 11A). No signal was detected in gut of carp intubated with control potato PAT4 material, showing that although the concentration of the applied GFP was higher than the potato derived LT-B-GFP, its uptake is far less efficient suggesting an additive effect of LT-B.

**Immunization of carp**. Potato tubers expressing LT-B-iipp (pL1516; approximately 5.3 micrograms of LT-B-iipp/g FW) and PAT4 were freeze dried, ground, homogenized and resuspended in PBS. Estimated amount of LT-B-iipp in freeze-dried pL1516 material is 21.2 micrograms/g DW. Fish (3 per group) were intubated with 300 µl potato pL1516 suspension (approximately 3 µg LT-B-iipp) One group of fish was killed 3 weeks post intubation and blood was collected for measurement primary response. A second group of fish was boostered anally after 8 weeks (secondary response) and killed 2 weeks post boostering, upon which blood samples were taken. Blood samples were allowed to cloth for 18 hours at 4°C, were centrifuged (10000 x g, 5 min. RT) and serum was collected.

Immune responses were measured as follows: Maxisorb ELISA plates (Nunc, Roskilde, Denmark) were coated for 18 hr at 4°C with 100 ml of 4 mg/ml anti-parvo peptide monoclonal antibody (3C9, Ingenaza, Madrid, Spain) followed by blocking with 0.5% BSA (Roche, Mannheim, Germany) for two hours at RT. Subsequently a synthetic peptide representing the amino-terminal region of the viral VP2 protein of canine parvovirus (CPV; Langeveld et al., 1994) was added. Sera were added and serially diluted. Detection was with biotin linked monoclonal antibody recognising carp serum Ig (WCI-12). Samples were diluted in PBS containing 0.5% BSA and 0.1% Tween 20 (Merck) and bound WCI-12 was visualized using streptavidin linked HRP (Sanquin, Amsterdam, The Netherlands, dilution: 1:5,000) and TMB peroxidase substrate (Kirkegaard & Perry, Gaithersburg, MD). The substrate was incubated for a maximum of 20 min, and subsequently the OD was measured at 450 nm. Anti parvo titres (pL1516 immunized fish) were defined by the dilution of the sample at an OD of 0.1. Anti-parvo responses could be detected in both primary and secondary immune serum by ELISA and results are summarized in Figure 12. Figure 12 shows that the titres measured in the sera of fish in the pL1516 (LT-B-iipp) treated group is on average higher compared to the control group showing that upon anal intubation antigen specific systemic humoral immune responses are induced.

In another experiment, carp were fed during 5 consecutive days with the pL2003 containing feed and PAT4 at a daily ration of 6% of their body weight. Four weeks after the last immunisation, sera were isolated and treated as described above (primary response). Eight weeks after the last immunisation fish were boostered by feeding them for one day at a ration of 4% of their body weight. Four weeks after the booster serum was isolated and treated as described above. As a positive control carp were both primed and boostered with 50 µg of purified recGFP in incomplete freunds adjuvant by intraperitoneal injection. For detection of anti-GFP immune responses, Maxisorb ELISA plates (Nunc) were coated for 18 hr at 4°C with 100 ml of 0.5 mg/ml anti-GFP antibody (ab1218, Abcam, Cambridge, UK) followed by blocking with 0.5% BSA (Roche) for two hours at RT. Subsequently 100 µl recGFP (1 mg/ml) was added and sera were added at serial dilutions. Anti-GFP specific serum antibodies were detected using biotin linked mAb recognising carp serum Ig (WCI-12). Samples were diluted in PBS containing 0.5% BSA and 0.1% Tween 20 (Merck). Finally WCI-12 was detected using streptavidin linked HRP (Sanquin, Amsterdam, The Netherlands, dilution: 1:5,000) and TMB peroxidase substrate (Kirkegaard & Perry, Gaithersburg, MD). The substrate was incubated for a maximum of 20 min, and subsequently the OD was measured at 450 nm. Anti GFP titres were defined by the dilution of the sample at an OD of 0.05 (primary response) or 0.2 (secondary response). Results are summarized in Figure 13 and clearly show that in secondary immune sera of carp fed with the pL2003 containing feed pellets, an elevated anti-GFP titre was detected (Figure 13) whereas control pellets did not evoke a GFP specific humoral immune response. These data show that oral administration of LT-B-GFP evokes an systemic humoral immune response.

### REFERENCES

Aitken, R., Hirst, T.R. (1993) Recombinant enterotoxins as vaccines against Escherichia coli-mediated diarrhea. Vaccine 11: 227-233.

Cardenas, L., Clements, J.D. (1993) Development of mucosal protection against the heat-stable enterotoxin (ST) of Escherichia coli by oral immunization with a genetic fusion delivered by a bacterial vector. Infection and Immunity 61: 4629-4636.

Dalmo, R.A., Ingebrigtsen, K., Bgwald, J. (1997) Non-specific defense mechanisms in fish, with particular reference to the reticuloendothelial system (RES). Journal of Fish Diseases 20: 241-273.

De Haan L, Verweij WR, Feil IK, Holtrop M, Hol WG, Agsteribbe E, et al. Role of GM1 binding in the mucosal immunogenicity and adjuvant activity of the Escherichia coli heat-labile enterotoxin and its B subunit. Immunology 1998;94(3):424-30.

Hart S., Wrathmell A.B., Harris J.E. and Grayson T.H. (1988) Gut immunology in fish: a review. Developmental and Comparative Immunology 12: 453-480.

Jagusztyn-Krynicka, E.K., Clark-Curtiss, J.E., Curtiss III, R. (1993) Escherichia coli heat-labile toxin subunit B fusions with Streptococcus sobrinus antigens expressed by Salmonella typhimurium oral vaccine strains: importance of the linker for antigeneicity and biological activities of the hybrid proteins. Infection and Immunity 61: 1004-1015.

Khoury, C.A., Meinersmann, R.J. (1995) A genetic hybrid of the Campylobacter jejuni flaA gene with LT-B of Escherichia coli and assessment of the efficacy of the hybrid protein as an oral chicken vaccine. Avian Diseases 39: 812-820.

Lamers C.H.J., (1985) The reaction of the immune system of fish to vaccination. PhD the-sis, Agricultural University of Wageningen.

Leong, J.C., Fryer, J.L. (1993) Viral vaccines for aquaculture. Annual Review of Fish Diseases 3: 225-240.

Lorenzen, N., Olesen, N.J. (1997) Immunisation with viral antigens: viral haemorrhagic septicaemia. In: Fish Vaccinology. Gudding, R., Lillehaug, A., Midlyng, P.J., Brown, F. (eds). Dev. Biol. Stand. Basel, Karger, vol. 90, p 201-209..

Lorenzen, N., Olesen, N.J., Koch, C. (1999) Immunity to VHS virus in rainbow trout. Aqua-culture 172: 41-61.

Mowat, A.M. (1994) Oral tolerance and regulation of immunity to dietary antigens. In: Handbook of mucosal immunology (Ogra, P.C., Lamm, M.E., Mestecky, J., Strober, W., McGhee, J.R., Bienenstock, J., eds.) pp. 185-201. San Diego: Academic Press.

Newman, S.G. (1993) Bacterial vaccines for fish. Annual Reviews Fish Diseases 3: 145-185.

Rombout J.H.W.M., Lamers C.H.J., Helfrich M.H.. Dekker A. and Taverne-Thiele J.J. (1985) Uptake and transport of intact macromolecules in the intestinal epithelium of carp (Cyprinus carpio L.). Cell Tissue Research 239: 519-530.

Rombout, J.H.W.M., van den Berg A.A. (1989) Immunological importance of the second gut segment of carp. I. Uptake and processing of antigens by epithelial and macrophages. Journal of Fish Biology 35: 13-22.

Rombout, J.H.W.M., van den Berg A.A., van den Berg, C.T.G.A., Witte, P., Egberts, E. (1989) Immunological importance of the second gut segment of carp. III. Systemic and /or mucosal immune responses after immunization with soluble or particulate antigen. Journal of Fish Biology 35: 179-186.

Walker, R.I. (1994) New strategies for using mucosal vaccination to achieve more effective immunization. Vaccine 12: 387-400.

Weiner, H.L., Friedman, A., Miller, A., Khoury, S.J., Al-Sabbagh, A., Santos, L., Sayegh, M., Nussenblatt, R.B., Trentham, D.E., Hafler, D.A. (1994) Oral tolerance: Immunologic mechanisms and treatment of animal and human organ-specific autoimmune diseases by oral administration of autoantigens. Annual Review in Immunology 12: 809-837.

## Claims

1. Use of a protein complex comprising an antigen of interest fused to the B-subunit of *Vibrio cholerae* cholera toxin (CT-B), or *Escherichia coli* heat-labile enterotoxin (LT-B) for the manufacture of an oral fish vaccine.

2. Use according to claim 1, wherein said antigen is derived from a virus or micro-organism pathogenic to fish.

3. Use according to claim 2, wherein said virus or micro-organism pathogenic to fish is selected from the group consisting of infectious pancreatic necrosis virus (IPNV), striped jack nervous necrosis virus (SJNNV), infectious haematopoietic necrosis virus (IHNV), viral haemorrhagic septicaemia virus (VHSV), Pancreas Disease virus (SPDV), infectious salmon anaemia virus (ISAV), Spring Viraemia of Carp virus (SVCV), Koi Herpesvirus (KHV), *Flexibacter columnaris, Edwardsialla ictaluri, E. tarda, Piscirickettsia salmonis, Vibrio* spp and *Aeromonas* spp., *Yersinia ruckeri*, *Pasturella piscicida* and *Renibacterium salmoninarum*.

4. Use according to claim 3, wherein said antigen of interest is selected from the group consisting of the VP2-protein of IPNV, the glycoprotein of VHSV (VHSV-G) and the glycoprotein of SVCV (SVCV-G).

5. Use according to any one of claims 1 to 4, wherein said protein complex is produced in an edible host cell, preferably a plant cell, more preferably a tuberous plant cell.

6. Fish feed comprising a protein complex comprising an antigen of interest fused to the B-subunit of *Vibrio cholerae* cholera toxin (CT-B), or *Escherichia coli* heat-labile enterotoxin (LT-B).

7. Fish feed according to claim 6, wherein said antigen is derived from a virus or micro-organism pathogenic to fish, preferably wherein said antigen is derived from a fish pathogen selected from the group consisting of infectious pancreatic necrosis virus (IPNV), striped jack nervous necrosis virus (SJNNV), infectious haematopoietic necrosis virus (IHNV), viral haemorrhagic septicaemia virus (VHSV), Pancreas Disease virus (SPDV), infectious salmon anaemia virus (ISAV), Spring Viraemia of Carp virus (SVCV), Koi Herpesvirus (KHV), *Flexibacter columnaris, Edwardsialla ictaluri, E. tarda, Piscirickettsia salmonis, Vibrio* spp and *Aeromonas* spp., *Yersinia ruckeri, Pasturella piscicida* and *Renibacterium salmoninarum*.

8. A method for the expression of a protein of interest in a host cell, preferably a plant host cell, wherein said protein originates from an organism that has an optimal growth temperature that lies below the optimal growth temperature of said host cell and wherein said protein is expressed as a fusion protein with a B-subunit of *Vibrio cholerae* cholera toxin (CT-B) or *Escherichia coli* heat-labile enterotoxin (LT-B).

9. A method for immunizing fish, comprising the oral administration of a composition comprising a protein complex comprising an antigen of interest fused to the B-subunit of *Vibrio cholerae* cholera toxin (CT-B), or *Escherichia coli* heat-labile enterotoxin (LT-B).


### Annex to the application documents - subsequently filed sequences listing

SEQUENCE LISTING

<110>  Plant Research International B.V.

<120>  Oral vaccines for fish

<130>  P71710EP00

<140>  05075203.9
<141>  2005-01-26

<160>  22

<170>  PatentIn version 3.2

<210>  1
<211>  34
<212>  DNA
<213>  Artificial

<220>
<223>  Primer IPNV forward

<400>  1
caggggatcc catgaacaca aacaaggcaa ccgc                                    34


<210>  2
<211>  42
<212>  DNA
<213>  Artificial

<220>
<223>  Primer IPNV reverse

<400>  2
aaaaacccgg gagatctcat tacacctcag cgttgtctcc gc                          42


<210>  3
<211>  33
<212>  DNA
<213>  Artificial

<220>
<223>  Oligonucleotide VHSV G1

<400>  3
tctggtgtcg accagatcac tcaacgacct ccg                                    33


<210>  4
<211>  37
<212>  DNA
<213>  Artificial

<220>
<223>  Oligonucleotide VHSV G3

<400>  4
gatcgaagat ctaagtcatc agaccgtctg acttctg                                37


<210>  5
<211>  18
<212>  DNA
<213>  Artificial

<220>
<223>  Primer G-F

<400>  5
caggggatcc cagatcac                                                    18


<210>  6
<211>  28
<212>  DNA
<213>  Artificial

<220>
<223>  Primer Glong-R-SmaI

<400>  6
aaaaacccgg gagatctcat taaagttc                                         28


<210>  7
<211>  29
<212>  DNA
<213>  Artificial

<220>
<223>  Oligonucleotide GFPFw

<400>  7
aggggatccg gcttccaagg gagaggaac                                        29


<210>  8
<211>  32
<212>  DNA
<213>  Artificial

<220>
<223>  Oligonucleotide GFPRev

<400>  8
ctcggatcct tcttgtacaa ctcatccatg cc                                    32


<210>  9
<211>  32
<212>  DNA
<213>  Artificial

<220>
<223>  Primer SVCVG1

<400>  9
tctggtctcg agatccccat atttgttcca tc                                    32


<210>  10
<211>  40
<212>  DNA
<213>  Artificial

<220>
<223>  Primer SVCVG2

<400>  10
gatcgaggat ccaagtcatc aaactaaaga ccgcatttcg                            40


<210>  11

<211>  34
<212>  DNA
<213>  Artificial

<220>
<223>  Primer LTBsal

<400>  11
gagtcgtcga cacctggagc gtagttcttc atgc                                    34


<210>  12
<211>  37
<212>  DNA
<213>  Artificial

<220>
<223>  Primer LTBnco

<400>  12
gtgacgaaga caacatgaac aaggtgaagt gttatgt                                 37


<210>  13
<211>  448
<212>  DNA
<213>  Escherichia coli


<220>
<221>  CDS
<222>  (17)..(424)
<223>  /note="Four restriction enzym sites (position 1-6, 10-15, 400-405
       and 437-442)"

<400>  13
gaattcgggg ttaaca atg aac aag gtg aag tgt tat gtg ttg ttc act gca      52
                  Met Asn Lys Val Lys Cys Tyr Val Leu Phe Thr Ala
                  1               5                   10

tta cta tcc tct cta tat gca cat ggt gct cct cag aca atc aca gaa      100
Leu Leu Ser Ser Leu Tyr Ala His Gly Ala Pro Gln Thr Ile Thr Glu
        15                  20                  25

ctt tgt tcc gaa tat cgc aac aca cag atc tac aca atc aat gac aag      148
Leu Cys Ser Glu Tyr Arg Asn Thr Gln Ile Tyr Thr Ile Asn Asp Lys
    30                  35                  40

atc ctt tca tac act gaa tct atg gca ggc aaa aga gaa atg gtg atc      196
Ile Leu Ser Tyr Thr Glu Ser Met Ala Gly Lys Arg Glu Met Val Ile
45                  50                  55                  60

atc aca ttc aag agc ggt gaa aca ttt caa gtc gaa gtc cca ggc agt      244
Ile Thr Phe Lys Ser Gly Glu Thr Phe Gln Val Glu Val Pro Gly Ser
                65                  70                  75

caa cac att gat tcc caa aag aag gcc att gaa agg atg aag gac acc      292
Gln His Ile Asp Ser Gln Lys Lys Ala Ile Glu Arg Met Lys Asp Thr
            80                  85                  90

ttg aga atc aca tat ctc aca gag acc aag att gac aag ttg tgt gtt      340
Leu Arg Ile Thr Tyr Leu Thr Glu Thr Lys Ile Asp Lys Leu Cys Val
        95                  100                 105

tgg aac aac aag act ccc aat tca att gct gca atc agc atg aag aac      388
Trp Asn Asn Lys Thr Pro Asn Ser Ile Ala Ala Ile Ser Met Lys Asn
    110                 115                 120

tac gct cca ggg gat ccg agt gaa aag gat gaa ctt taatgaagat 434
Tyr Ala Pro Gly Asp Pro Ser Glu Lys Asp Glu Leu
125                 130                 135

ctcccgggct taat 448

<210> 14
<211> 136
<212> PRT
<213> Escherichia coli

<400> 14

Met Asn Lys Val Lys Cys Tyr Val Leu Phe Thr Ala Leu Leu Ser Ser
1               5                   10                  15

Leu Tyr Ala His Gly Ala Pro Gln Thr Ile Thr Glu Leu Cys Ser Glu
            20                  25                  30

Tyr Arg Asn Thr Gln Ile Tyr Thr Ile Asn Asp Lys Ile Leu Ser Tyr
        35                  40                  45

Thr Glu Ser Met Ala Gly Lys Arg Glu Met Val Ile Ile Thr Phe Lys
    50                  55                  60

Ser Gly Glu Thr Phe Gln Val Glu Val Pro Gly Ser Gln His Ile Asp
65                  70                  75                  80

Ser Gln Lys Lys Ala Ile Glu Arg Met Lys Asp Thr Leu Arg Ile Thr
                85                  90                  95

Tyr Leu Thr Glu Thr Lys Ile Asp Lys Leu Cys Val Trp Asn Asn Lys
            100                 105                 110

Thr Pro Asn Ser Ile Ala Ala Ile Ser Met Lys Asn Tyr Ala Pro Gly
        115                 120                 125

Asp Pro Ser Glu Lys Asp Glu Leu
    130                 135

<210> 15
<211> 1915
<212> DNA
<213> Artificial

<220>
<223> Fusion protein LT-B + VHSVG

<220>
<221> CDS
<222> (50)..(1900)

<400> 15
   ccgctgagga gctccttcta cttgcacatg ttcgaattcg gggttaaca atg aac aag 58
   Met Asn Lys
   1
   gtg aag tgt tat gtg ttg ttc act gca tta cta tcc tct cta tat gca     106
   Val Lys Cys Tyr Val Leu Phe Thr Ala Leu Leu Ser Ser Leu Tyr Ala
       5                   10                  15
   cat ggt gct cct cag aca atc aca gaa ctt tgt tcc gaa tat cgc aac     154
   His Gly Ala Pro Gln Thr Ile Thr Glu Leu Cys Ser Glu Tyr Arg Asn
   20                  25                  30                  35
   aca cag atc tac aca atc aat gac aag atc ctt tca tac act gaa tct     202
   Thr Gln Ile Tyr Thr Ile Asn Asp Lys Ile Leu Ser Tyr Thr Glu Ser
                   40                  45                  50
   atg gca ggc aaa aga gaa atg gtg atc atc aca ttc aag agc ggt gaa     250
   Met Ala Gly Lys Arg Glu Met Val Ile Ile Thr Phe Lys Ser Gly Glu
               55                  60                  65
   aca ttt caa gtc gaa gtc cca ggc agt caa cac att gat tcc caa aag     298
   Thr Phe Gln Val Glu Val Pro Gly Ser Gln His Ile Asp Ser Gln Lys
           70                  75                  80
   aag gcc att gaa agg atg aag gac acc ttg aga atc aca tat ctc aca     346
   Lys Ala Ile Glu Arg Met Lys Asp Thr Leu Arg Ile Thr Tyr Leu Thr
       85                  90                  95
   gag acc aag att gac aag ttg tgt gtt tgg aac aac aag act ccc aat     394
   Glu Thr Lys Ile Asp Lys Leu Cys Val Trp Asn Asn Lys Thr Pro Asn
   100                 105                 110                 115
   tca att gct gca atc agc atg aag aac tac gct cca ggg gat ccg cag     442
   Ser Ile Ala Ala Ile Ser Met Lys Asn Tyr Ala Pro Gly Asp Pro Gln
                   120                 125                 130
   atc act caa cga cct ccg gtc gaa aac atc tcg acg tac cat gca gat     490
   Ile Thr Gln Arg Pro Pro Val Glu Asn Ile Ser Thr Tyr His Ala Asp
               135                 140                 145
   tgg gac act ccg cta tac act cat ccc tcc aac tgc agg gac gat tcc     538
   Trp Asp Thr Pro Leu Tyr Thr His Pro Ser Asn Cys Arg Asp Asp Ser
           150                 155                 160
   ttt gtc ccg att cga cca gct caa ctc agg tgt cct cat gaa ttt gaa     586
   Phe Val Pro Ile Arg Pro Ala Gln Leu Arg Cys Pro His Glu Phe Glu
       165                 170                 175
   gac ata aac aag gga ctg gtc tcc gtc cca acc aag atc atc cat ctc     634
   Asp Ile Asn Lys Gly Leu Val Ser Val Pro Thr Lys Ile Ile His Leu
   180                 185                 190                 195
   ccg cta tca gtc acc agc gtc tcc gca gtc gcg agt ggc cac tac ctg     682
   Pro Leu Ser Val Thr Ser Val Ser Ala Val Ala Ser Gly His Tyr Leu
                   200                 205                 210
   cac aga gtg act tat cga gtc acc tgt tcg acc agc ttc ttt gga ggg     730
   His Arg Val Thr Tyr Arg Val Thr Cys Ser Thr Ser Phe Phe Gly Gly
               215                 220                 225
   caa acc att gaa aag acc atc tta gag gcg aaa ctg tct cgt cag gag     778
   Gln Thr Ile Glu Lys Thr Ile Leu Glu Ala Lys Leu Ser Arg Gln Glu
           230                 235                 240
   gcc aca gac gag gca agc aag gat cac gag tac ccg ttc ttc cct gaa     826
   Ala Thr Asp Glu Ala Ser Lys Asp His Glu Tyr Pro Phe Phe Pro Glu
       245                 250                 255
   ccc tcc tgc atc tgg atg aaa aac aat gtc cat aag gac ata act cac     874
   Pro Ser Cys Ile Trp Met Lys Asn Asn Val His Lys Asp Ile Thr His
   260                 265                 270                 275
   tat tac aag acc cca aaa aca gta tcg gtg gat ctc tac agc agg aaa 922
   Tyr Tyr Lys Thr Pro Lys Thr Val Ser Val Asp Leu Tyr Ser Arg Lys
   280 285 290
   ttt ctc aac cct gat ttc ata gag ggg gtt tgc aca acc tcg ccc tgt 970
   Phe Leu Asn Pro Asp Phe Ile Glu Gly Val Cys Thr Thr Ser Pro Cys
   295 300 305
   caa act cat tgg cag gga gtc tat tgg gtc ggt gcc aca ccc aaa gcc 1018
   Gln Thr His Trp Gln Gly Val Tyr Trp Val Gly Ala Thr Pro Lys Ala
   310 315 320
   cat tgc ccc acg tcg gaa aca cta gaa gga cac ctg ttc acc agg acc 1066
   His Cys Pro Thr Ser Glu Thr Leu Glu Gly His Leu Phe Thr Arg Thr
   325 330 335
   cat gat cac agg gtg gtc aag gca att gtg gca ggc cat cat ccc tgg 1114
   His Asp His Arg Val Val Lys Ala Ile Val Ala Gly His His Pro Trp
   340 345 350 355
   gga ctc aca atg gca tgc aca gtg aca ttc tgc ggg gca gaa tgg atc 1162
   Gly Leu Thr Met Ala Cys Thr Val Thr Phe Cys Gly Ala Glu Trp Ile
   360 365 370
   aag act gac ctg gga gac ctg atc cag gta aca gga ccg ggg ggc acg 1210
   Lys Thr Asp Leu Gly Asp Leu Ile Gln Val Thr Gly Pro Gly Gly Thr
   375 380 385
   ggg aaa ctg act cca aag aag tgt gtc aat gct gat gtc cag atg agg 1258
   Gly Lys Leu Thr Pro Lys Lys Cys Val Asn Ala Asp Val Gln Met Arg
   390 395 400
   ggg gca aca gat gac ttt tct tat ctc aac cat ctc atc acc aac atg 1306
   Gly Ala Thr Asp Asp Phe Ser Tyr Leu Asn His Leu Ile Thr Asn Met
   405 410 415
   gct caa aga acc gag tgc cta gat gcc cat agt gat atc acc gct tct 1354
   Ala Gln Arg Thr Glu Cys Leu Asp Ala His Ser Asp Ile Thr Ala Ser
   420 425 430 435
   ggg aaa ata tcc tca ttt ctc ctc tca aag ttt cgt ccc agc cac cct 1402
   Gly Lys Ile Ser Ser Phe Leu Leu Ser Lys Phe Arg Pro Ser His Pro
   440 445 450
   gga ccc ggc aag gca cac tat ctt ctc aac ggt caa atc atg cga ggt 1450
   Gly Pro Gly Lys Ala His Tyr Leu Leu Asn Gly Gln Ile Met Arg Gly
   455 460 465
   gac tgt gac tat gag gca gta gtc agc atc aac tac aac agc gct caa 1498
   Asp Cys Asp Tyr Glu Ala Val Val Ser Ile Asn Tyr Asn Ser Ala Gln
   470 475 480
   tac aag aca gtg aac aac aca tgg aaa tca tgg aaa cgg gta gac aac 1546
   Tyr Lys Thr Val Asn Asn Thr Trp Lys Ser Trp Lys Arg Val Asp Asn
   485 490 495
   aac aca gac ggg tac gat ggg atg ata ttt ggg gac aaa ttg atc atc 1594
   Asn Thr Asp Gly Tyr Asp Gly Met Ile Phe Gly Asp Lys Leu Ile Ile
   500 505 510 515
   ccg gac atc gag aag tat cag agt gtc tat gac agt gga atg ctc gtt 1642
   Pro Asp Ile Glu Lys Tyr Gln Ser Val Tyr Asp Ser Gly Met Leu Val
   520 525 530
   caa aga aac ctt gtg gaa gtc cct cat ctg agc acc gtg ttt gtc tcc 1690
   Gln Arg Asn Leu Val Glu Val Pro His Leu Ser Thr Val Phe Val Ser
   535 540 545
   aac aca tcc gat ctt tcc act aat cac atc cac acc aac cta atc cct 1738
   Asn Thr Ser Asp Leu Ser Thr Asn His Ile His Thr Asn Leu Ile Pro
   550 555 560
   tcg gat tgg tca ttc cac tgg agt att tgg ccc tca tta tct ggg atg 1786
   Ser Asp Trp Ser Phe His Trp Ser Ile Trp Pro Ser Leu Ser Gly Met
   565 570 575
   ggg gtt gtg gga ggg gcc ttc ctt cta ctg gta ctc tgc tgt tgc tgc 1834
   Gly Val Val Gly Gly Ala Phe Leu Leu Leu Val Leu Cys Cys Cys Cys
   580 585 590 595
   aag gcg tcc cct ccc att cca aat tac ggg att ccg atg cag cag ttc 1882
   Lys Ala Ser Pro Pro Ile Pro Asn Tyr Gly Ile Pro Met Gln Gln Phe
   600 605 610
   tcc aga agt cag acg gtc tgatgactta gatct 1915
   Ser Arg Ser Gln Thr Val
   615

<210> 16
<211> 617
<212> PRT
<213> Artificial

<220>
<223> Synthetic Construct

<400> 16

Met Asn Lys Val Lys Cys Tyr Val Leu Phe Thr Ala Leu Leu Ser Ser
1               5                   10                  15

Leu Tyr Ala His Gly Ala Pro Gln Thr Ile Thr Glu Leu Cys Ser Glu
            20                  25                  30

Tyr Arg Asn Thr Gln Ile Tyr Thr Ile Asn Asp Lys Ile Leu Ser Tyr
        35                  40                  45

Thr Glu Ser Met Ala Gly Lys Arg Glu Met Val Ile Ile Thr Phe Lys
    50                  55                  60

Ser Gly Glu Thr Phe Gln Val Glu Val Pro Gly Ser Gln His Ile Asp
65                  70                  75                  80

Ser Gln Lys Lys Ala Ile Glu Arg Met Lys Asp Thr Leu Arg Ile Thr
                85                  90                  95

Tyr Leu Thr Glu Thr Lys Ile Asp Lys Leu Cys Val Trp Asn Asn Lys
            100                 105                 110

Thr Pro Asn Ser Ile Ala Ala Ile Ser Met Lys Asn Tyr Ala Pro Gly
        115                 120                 125

Asp Pro Gln Ile Thr Gln Arg Pro Pro Val Glu Asn Ile Ser Thr Tyr
    130                 135                 140

His Ala Asp Trp Asp Thr Pro Leu Tyr Thr His Pro Ser Asn Cys Arg

145                 150                 155                 160

Asp Asp Ser Phe Val Pro Ile Arg Pro Ala Gln Leu Arg Cys Pro His
                165                 170                 175

Glu Phe Glu Asp Ile Asn Lys Gly Leu Val Ser Val Pro Thr Lys Ile
            180                 185                 190

Ile His Leu Pro Leu Ser Val Thr Ser Val Ser Ala Val Ala Ser Gly
        195                 200                 205

His Tyr Leu His Arg Val Thr Tyr Arg Val Thr Cys Ser Thr Ser Phe
    210                 215                 220

Phe Gly Gly Gln Thr Ile Glu Lys Thr Ile Leu Glu Ala Lys Leu Ser
225                 230                 235                 240

Arg Gln Glu Ala Thr Asp Glu Ala Ser Lys Asp His Glu Tyr Pro Phe
                245                 250                 255

Phe Pro Glu Pro Ser Cys Ile Trp Met Lys Asn Asn Val His Lys Asp
            260                 265                 270

Ile Thr His Tyr Tyr Lys Thr Pro Lys Thr Val Ser Val Asp Leu Tyr
        275                 280                 285

Ser Arg Lys Phe Leu Asn Pro Asp Phe Ile Glu Gly Val Cys Thr Thr
    290                 295                 300

Ser Pro Cys Gln Thr His Trp Gln Gly Val Tyr Trp Val Gly Ala Thr
305                 310                 315                 320

Pro Lys Ala His Cys Pro Thr Ser Glu Thr Leu Glu Gly His Leu Phe
                325                 330                 335

Thr Arg Thr His Asp His Arg Val Val Lys Ala Ile Val Ala Gly His
            340                 345                 350

His Pro Trp Gly Leu Thr Met Ala Cys Thr Val Thr Phe Cys Gly Ala
        355                 360                 365

Glu Trp Ile Lys Thr Asp Leu Gly Asp Leu Ile Gln Val Thr Gly Pro
    370                 375                 380

Gly Gly Thr Gly Lys Leu Thr Pro Lys Lys Cys Val Asn Ala Asp Val
385                 390                 395                 400

Gln Met Arg Gly Ala Thr Asp Asp Phe Ser Tyr Leu Asn His Leu Ile
                405                 410                 415

Thr Asn Met Ala Gln Arg Thr Glu Cys Leu Asp Ala His Ser Asp Ile

420                 425                 430

Thr Ala Ser Gly Lys Ile Ser Ser Phe Leu Leu Ser Lys Phe Arg Pro
        435                 440                 445

Ser His Pro Gly Pro Gly Lys Ala His Tyr Leu Leu Asn Gly Gln Ile
    450                 455                 460

Met Arg Gly Asp Cys Asp Tyr Glu Ala Val Val Ser Ile Asn Tyr Asn
465                 470                 475                 480

Ser Ala Gln Tyr Lys Thr Val Asn Asn Thr Trp Lys Ser Trp Lys Arg
                485                 490                 495

Val Asp Asn Asn Thr Asp Gly Tyr Asp Gly Met Ile Phe Gly Asp Lys
            500                 505                 510

Leu Ile Ile Pro Asp Ile Glu Lys Tyr Gln Ser Val Tyr Asp Ser Gly
        515                 520                 525

Met Leu Val Gln Arg Asn Leu Val Glu Val Pro His Leu Ser Thr Val
    530                 535                 540

Phe Val Ser Asn Thr Ser Asp Leu Ser Thr Asn His Ile His Thr Asn
545                 550                 555                 560

Leu Ile Pro Ser Asp Trp Ser Phe His Trp Ser Ile Trp Pro Ser Leu
                565                 570                 575

Ser Gly Met Gly Val Val Gly Gly Ala Phe Leu Leu Leu Val Leu Cys
            580                 585                 590

Cys Cys Cys Lys Ala Ser Pro Pro Ile Pro Asn Tyr Gly Ile Pro Met
        595                 600                 605

Gln Gln Phe Ser Arg Ser Gln Thr Val
    610                 615

<210>  17
<211>  1155
<212>  DNA
<213>  Artificial

<220>
<223>  Fusion protein LT-B + GFP


<220>
<221>  CDS
<222>  (1)..(1131)

<400>  17
atg aac aag gtg aag tgt tat gtg ttg ttc act gca tta cta tcc tct      48
Met Asn Lys Val Lys Cys Tyr Val Leu Phe Thr Ala Leu Leu Ser Ser
1               5                   10                  15

cta tat gca cat ggt gct cct cag aca atc aca gaa ctt tgt tcc gaa 96
Leu Tyr Ala His Gly Ala Pro Gln Thr Ile Thr Glu Leu Cys Ser Glu
            20                  25                  30

tat cgc aac aca cag atc tac aca atc aat gac aag atc ctt tca tac 144
Tyr Arg Asn Thr Gln Ile Tyr Thr Ile Asn Asp Lys Ile Leu Ser Tyr
        35                  40                  45

act gaa tct atg gca ggc aaa aga gaa atg gtg atc atc aca ttc aag 192
Thr Glu Ser Met Ala Gly Lys Arg Glu Met Val Ile Ile Thr Phe Lys
    50                  55                  60

agc ggt gaa aca ttt caa gtc gaa gtc cca ggc agt caa cac att gat 240
Ser Gly Glu Thr Phe Gln Val Glu Val Pro Gly Ser Gln His Ile Asp
65                  70                  75                  80

tcc caa aag aag gcc att gaa agg atg aag gac acc ttg aga atc aca 288
Ser Gln Lys Lys Ala Ile Glu Arg Met Lys Asp Thr Leu Arg Ile Thr
                85                  90                  95

tat ctc aca gag acc aag att gac aag ttg tgt gtt tgg aac aac aag 336
Tyr Leu Thr Glu Thr Lys Ile Asp Lys Leu Cys Val Trp Asn Asn Lys
            100                 105                 110

act ccc aat tca att gct gca atc agc atg aag aac tac gct cca ggg 384
Thr Pro Asn Ser Ile Ala Ala Ile Ser Met Lys Asn Tyr Ala Pro Gly
        115                 120                 125

gat ccg gct tcc aag gga gag gaa ctt ttc act gga gtt gtc cca atc 432
Asp Pro Ala Ser Lys Gly Glu Glu Leu Phe Thr Gly Val Val Pro Ile
    130                 135                 140

ttg gtt gag ctc gac ggt gac gtc aat gga cac aag ttt tcc gtc tca 480
Leu Val Glu Leu Asp Gly Asp Val Asn Gly His Lys Phe Ser Val Ser
145                 150                 155                 160

gga gag ggt gaa ggt gat gca acc tac gga aag ttg acc ctt aag ttc 528
Gly Glu Gly Glu Gly Asp Ala Thr Tyr Gly Lys Leu Thr Leu Lys Phe
                165                 170                 175

atc tgc act act gga aaa ctc cct gtt cct tgg cca aca ttg gtg acc 576
Ile Cys Thr Thr Gly Lys Leu Pro Val Pro Trp Pro Thr Leu Val Thr
            180                 185                 190

act ttg act tac ggt gtt caa tgc ttc tca cgt tac cca gac cat atg 624
Thr Leu Thr Tyr Gly Val Gln Cys Phe Ser Arg Tyr Pro Asp His Met
        195                 200                 205

aag cgt cat gac ttt ttc aag tcc gcc atg ccc gag ggt tat gtg caa 672
Lys Arg His Asp Phe Phe Lys Ser Ala Met Pro Glu Gly Tyr Val Gln
    210                 215                 220

gag cgt act atc ttc ttc aag gac gac gga aac tac aag aca cgt gcc 720
Glu Arg Thr Ile Phe Phe Lys Asp Asp Gly Asn Tyr Lys Thr Arg Ala
225                 230                 235                 240

gaa gtc aag ttc gaa ggt gac acc ttg gtg aac aga atc gag ttg aag 768
Glu Val Lys Phe Glu Gly Asp Thr Leu Val Asn Arg Ile Glu Leu Lys
                245                 250                 255

ggt atc gat ttc aag gag gac gga aac att ctt gga cac aag ctt gag 816
Gly Ile Asp Phe Lys Glu Asp Gly Asn Ile Leu Gly His Lys Leu Glu
            260                 265                 270

tac aac tac aac tca cac aat gtg tac atc atg gct gac aag cag aag 864
Tyr Asn Tyr Asn Ser His Asn Val Tyr Ile Met Ala Asp Lys Gln Lys
        275                 280                 285

aac gga atc aag gtt aac ttc aaa atc cgt cac aac att gag gat gga 912
Asn Gly Ile Lys Val Asn Phe Lys Ile Arg His Asn Ile Glu Asp Gly
    290                 295                 300

agc gtt cag ttg gct gat cac tac caa cag aat act cca att ggc gat 960
Ser Val Gln Leu Ala Asp His Tyr Gln Gln Asn Thr Pro Ile Gly Asp
305                 310                 315                 320

ggc cct gtg ctt ttg cca gac aac cac tac ttg tcc acc caa tct gcc 1008
Gly Pro Val Leu Leu Pro Asp Asn His Tyr Leu Ser Thr Gln Ser Ala
                325                 330                 335

ctt tcc aaa gat ccc aac gaa aag aga gac cac atg gtc ttg ctt gag 1056
Leu Ser Lys Asp Pro Asn Glu Lys Arg Asp His Met Val Leu Leu Glu
            340                 345                 350

ttt gtg acc gct gct ggc att acc cac ggc atg gat gag ttg tac aag 1104
Phe Val Thr Ala Ala Gly Ile Thr His Gly Met Asp Glu Leu Tyr Lys
        355                 360                 365

aag gat ccg agt gaa aag gat gaa ctt taatgaagat ctcccgggct taat 1155
Lys Asp Pro Ser Glu Lys Asp Glu Leu
    370                 375

<210> 18
<211> 377
<212> PRT
<213> Artificial

<220>
<223> Synthetic Construct

<400> 18

Met Asn Lys Val Lys Cys Tyr Val Leu Phe Thr Ala Leu Leu Ser Ser
1               5                   10                  15

Leu Tyr Ala His Gly Ala Pro Gln Thr Ile Thr Glu Leu Cys Ser Glu
            20                  25                  30

Tyr Arg Asn Thr Gln Ile Tyr Thr Ile Asn Asp Lys Ile Leu Ser Tyr
        35                  40                  45

Thr Glu Ser Met Ala Gly Lys Arg Glu Met Val Ile Ile Thr Phe Lys
    50                  55                  60

Ser Gly Glu Thr Phe Gln Val Glu Val Pro Gly Ser Gln His Ile Asp
65                  70                  75                  80

Ser Gln Lys Lys Ala Ile Glu Arg Met Lys Asp Thr Leu Arg Ile Thr
                85                  90                  95

Tyr Leu Thr Glu Thr Lys Ile Asp Lys Leu Cys Val Trp Asn Asn Lys
            100                 105                 110

Thr Pro Asn Ser Ile Ala Ala Ile Ser Met Lys Asn Tyr Ala Pro Gly
        115                 120                 125

Asp Pro Ala Ser Lys Gly Glu Glu Leu Phe Thr Gly Val Val Pro Ile
    130                 135                 140

Leu Val Glu Leu Asp Gly Asp Val Asn Gly His Lys Phe Ser Val Ser
145                 150                 155                 160

Gly Glu Gly Glu Gly Asp Ala Thr Tyr Gly Lys Leu Thr Leu Lys Phe
                165                 170                 175

Ile Cys Thr Thr Gly Lys Leu Pro Val Pro Trp Pro Thr Leu Val Thr
            180                 185                 190

Thr Leu Thr Tyr Gly Val Gln Cys Phe Ser Arg Tyr Pro Asp His Met
        195                 200                 205

Lys Arg His Asp Phe Phe Lys Ser Ala Met Pro Glu Gly Tyr Val Gln
    210                 215                 220

Glu Arg Thr Ile Phe Phe Lys Asp Asp Gly Asn Tyr Lys Thr Arg Ala
225                 230                 235                 240

Glu Val Lys Phe Glu Gly Asp Thr Leu Val Asn Arg Ile Glu Leu Lys
                245                 250                 255

Gly Ile Asp Phe Lys Glu Asp Gly Asn Ile Leu Gly His Lys Leu Glu
            260                 265                 270

Tyr Asn Tyr Asn Ser His Asn Val Tyr Ile Met Ala Asp Lys Gln Lys
        275                 280                 285

Asn Gly Ile Lys Val Asn Phe Lys Ile Arg His Asn Ile Glu Asp Gly
    290                 295                 300

Ser Val Gln Leu Ala Asp His Tyr Gln Gln Asn Thr Pro Ile Gly Asp
305                 310                 315                 320

Gly Pro Val Leu Leu Pro Asp Asn His Tyr Leu Ser Thr Gln Ser Ala
                325                 330                 335

Leu Ser Lys Asp Pro Asn Glu Lys Arg Asp His Met Val Leu Leu Glu
            340                 345                 350

Phe Val Thr Ala Ala Gly Ile Thr His Gly Met Asp Glu Leu Tyr Lys
        355                 360                 365

Lys Asp Pro Ser Glu Lys Asp Glu Leu
    370                 375

<210> 19
<211> 1539
<212> DNA
<213> Spring viremia of carp virus

<220>
<221>  CDS
<222>  (1)..(1536)
<223>  /note="SVCV-G"

<400>  19
atg ggc atc aag atg gag aca cat tct cag gtc ttt gta tac atg ttg      48
Met Gly Ile Lys Met Glu Thr His Ser Gln Val Phe Val Tyr Met Leu
1               5                   10                  15

ctg tgg ttg tct ggt gtc gag ata ttt gtt cca tct ggt cga aat ata      96
Leu Trp Leu Ser Gly Val Glu Ile Phe Val Pro Ser Gly Arg Asn Ile
            20                  25                  30

tca tgg cag cct gtg att cag cca ttt gat tat caa tgt cca atc cat      144
Ser Trp Gln Pro Val Ile Gln Pro Phe Asp Tyr Gln Cys Pro Ile His
        35                  40                  45

gga aac cta ccc aac acg atg ggt ttg agt gcc acc aaa ttg aca ata      192
Gly Asn Leu Pro Asn Thr Met Gly Leu Ser Ala Thr Lys Leu Thr Ile
    50                  55                  60

aaa tct cca tct gta ttc agt aca gat aaa gtg tct gga tgg atc tgc      240
Lys Ser Pro Ser Val Phe Ser Thr Asp Lys Val Ser Gly Trp Ile Cys
65                  70                  75                  80

cat gca gct gaa tgg aaa aca act tgt gat tat aga tgg tat gga ccc      288
His Ala Ala Glu Trp Lys Thr Thr Cys Asp Tyr Arg Trp Tyr Gly Pro
                85                  90                  95

caa tac atc acc cac agt att cat ccg atc agt cct acc ata gat gaa      336
Gln Tyr Ile Thr His Ser Ile His Pro Ile Ser Pro Thr Ile Asp Glu
            100                 105                 110

tgc agg aga atc att caa agg att gca tca ggg act gat gaa gat ctg      384
Cys Arg Arg Ile Ile Gln Arg Ile Ala Ser Gly Thr Asp Glu Asp Leu
        115                 120                 125

ggg ttt ccc cct caa agt tgc gga tgg gca tct gtc aca acg gtg tca      432
Gly Phe Pro Pro Gln Ser Cys Gly Trp Ala Ser Val Thr Thr Val Ser
    130                 135                 140

aat act aat tat aga gta gta ccc cat tct gtt cat tta gag cca tac      480
Asn Thr Asn Tyr Arg Val Val Pro His Ser Val His Leu Glu Pro Tyr
145                 150                 155                 160

gga gga cat tgg atc gat cac gaa ttt aat ggg ggc gaa tgc aga gaa      528
Gly Gly His Trp Ile Asp His Glu Phe Asn Gly Gly Glu Cys Arg Glu
                165                 170                 175

aaa gtg tgc gaa atg aaa ggg aat cac tct att tgg atc aca gag gag      576
Lys Val Cys Glu Met Lys Gly Asn His Ser Ile Trp Ile Thr Glu Glu
            180                 185                 190

act gta cag cac gag tgt gca aaa cat ata gag gaa gtt gaa gga atc      624
Thr Val Gln His Glu Cys Ala Lys His Ile Glu Glu Val Glu Gly Ile
        195                 200                 205

atg tac ggg aat gtt cca aga ggg gat gta atg tat gct aac aac ttc      672
Met Tyr Gly Asn Val Pro Arg Gly Asp Val Met Tyr Ala Asn Asn Phe
    210                 215                 220

att ata gat aga cat cat aga gta tac aga ttc ggg gga tct tgt cag      720
Ile Ile Asp Arg His His Arg Val Tyr Arg Phe Gly Gly Ser Cys Gln
225                 230                 235                 240

atg aaa ttc tgt aat aaa gat ggt ata aaa ttc gcg aga gga gac tgg 768
Met Lys Phe Cys Asn Lys Asp Gly Ile Lys Phe Ala Arg Gly Asp Trp
                245                 250                 255

gta gag aaa aca gcc gga aca tta aca acg att cat gac aat gtg cct 816
Val Glu Lys Thr Ala Gly Thr Leu Thr Thr Ile His Asp Asn Val Pro
            260                 265                 270

aaa tgt gtt gat gga acg ttg gtc tcc ggt cac cgc ccc gga tta gac 864
Lys Cys Val Asp Gly Thr Leu Val Ser Gly His Arg Pro Gly Leu Asp
        275                 280                 285

ttg att gac aca atc ttc aat ttg gaa aat gta gtg gaa tat act ttg 912
Leu Ile Asp Thr Ile Phe Asn Leu Glu Asn Val Val Glu Tyr Thr Leu
    290                 295                 300

tgt gaa ggg act aaa agg aaa atc aat aaa caa gag aag ctg aca tca 960
Cys Glu Gly Thr Lys Arg Lys Ile Asn Lys Gln Glu Lys Leu Thr Ser
305                 310                 315                 320

gtg gat ttg agc tat ttg gct cca aga atc gga ggg ttc gga tca gta 1008
Val Asp Leu Ser Tyr Leu Ala Pro Arg Ile Gly Gly Phe Gly Ser Val
                325                 330                 335

ttt aga gtc aga aac gga aca tta gag aga ggg agc act act tat atc 1056
Phe Arg Val Arg Asn Gly Thr Leu Glu Arg Gly Ser Thr Thr Tyr Ile
            340                 345                 350

agg atc gaa gta gag gga cct att gtc gac tcg tta aat ggg aca gac 1104
Arg Ile Glu Val Glu Gly Pro Ile Val Asp Ser Leu Asn Gly Thr Asp
        355                 360                 365

cct aga act aac gcc tca aga gta ttc tgg gat gac tgg gag tta gat 1152
Pro Arg Thr Asn Ala Ser Arg Val Phe Trp Asp Asp Trp Glu Leu Asp
    370                 375                 380

ggc aat ata tac cag ggt ttt aat ggt gta tat aaa ggg aaa gat ggg 1200
Gly Asn Ile Tyr Gln Gly Phe Asn Gly Val Tyr Lys Gly Lys Asp Gly
385                 390                 395                 400

aag atc cat atc cct tta aat atg ata gaa tca gga atc ata gat aat 1248
Lys Ile His Ile Pro Leu Asn Met Ile Glu Ser Gly Ile Ile Asp Asn
                405                 410                 415

gag ctt caa cat gct ttc caa gcc gat att atc cct cat cct cat tat 1296
Glu Leu Gln His Ala Phe Gln Ala Asp Ile Ile Pro His Pro His Tyr
            420                 425                 430

gac gac gat gaa atc cga gag gat gat ata ttt ttc gat aac act gga 1344
Asp Asp Asp Glu Ile Arg Glu Asp Asp Ile Phe Phe Asp Asn Thr Gly
        435                 440                 445

gaa aat gga aat cct gta gat gca gtg gta gaa tgg gtt agt gga tgg 1392
Glu Asn Gly Asn Pro Val Asp Ala Val Val Glu Trp Val Ser Gly Trp
    450                 455                 460

gga act agt cta aag ttc ttt ggc atg act ctg gtc gcc ctg atc atg 1440
Gly Thr Ser Leu Lys Phe Phe Gly Met Thr Leu Val Ala Leu Ile Met
465                 470                 475                 480

atc ttt ctt ctc att aga tgc tgt gtt gct tgc act tat ttg atg aag 1488
Ile Phe Leu Leu Ile Arg Cys Cys Val Ala Cys Thr Tyr Leu Met Lys
                485                 490                 495

agg agt aaa cgg cct gca aca gaa tca cac gaa atg cgg tct tta gtt 1536
Arg Ser Lys Arg Pro Ala Thr Glu Ser His Glu Met Arg Ser Leu Val
            500                 505                 510

tga                                                                    1539

<210>  20
<211>  512
<212>  PRT
<213>  Spring viremia of carp virus

<400>  20

Met Gly Ile Lys Met Glu Thr His Ser Gln Val Phe Val Tyr Met Leu
1               5                   10                  15

Leu Trp Leu Ser Gly Val Glu Ile Phe Val Pro Ser Gly Arg Asn Ile
            20                  25                  30

Ser Trp Gln Pro Val Ile Gln Pro Phe Asp Tyr Gln Cys Pro Ile His
        35                  40                  45

Gly Asn Leu Pro Asn Thr Met Gly Leu Ser Ala Thr Lys Leu Thr Ile
    50                  55                  60

Lys Ser Pro Ser Val Phe Ser Thr Asp Lys Val Ser Gly Trp Ile Cys
65                  70                  75                  80

His Ala Ala Glu Trp Lys Thr Thr Cys Asp Tyr Arg Trp Tyr Gly Pro
                85                  90                  95

Gln Tyr Ile Thr His Ser Ile His Pro Ile Ser Pro Thr Ile Asp Glu
            100                 105                 110

Cys Arg Arg Ile Ile Gln Arg Ile Ala Ser Gly Thr Asp Glu Asp Leu
        115                 120                 125

Gly Phe Pro Pro Gln Ser Cys Gly Trp Ala Ser Val Thr Thr Val Ser
    130                 135                 140

Asn Thr Asn Tyr Arg Val Val Pro His Ser Val His Leu Glu Pro Tyr
145                 150                 155                 160

Gly Gly His Trp Ile Asp His Glu Phe Asn Gly Gly Glu Cys Arg Glu
                165                 170                 175

Lys Val Cys Glu Met Lys Gly Asn His Ser Ile Trp Ile Thr Glu Glu
            180                 185                 190

Thr Val Gln His Glu Cys Ala Lys His Ile Glu Glu Val Glu Gly Ile
        195                 200                 205

Met Tyr Gly Asn Val Pro Arg Gly Asp Val Met Tyr Ala Asn Asn Phe
    210                 215                 220

Ile Ile Asp Arg His His Arg Val Tyr Arg Phe Gly Gly Ser Cys Gln
225                 230                 235                 240

Met Lys Phe Cys Asn Lys Asp Gly Ile Lys Phe Ala Arg Gly Asp Trp
                245                 250                 255

Val Glu Lys Thr Ala Gly Thr Leu Thr Thr Ile His Asp Asn Val Pro
            260                 265                 270

Lys Cys Val Asp Gly Thr Leu Val Ser Gly His Arg Pro Gly Leu Asp
        275                 280                 285

Leu Ile Asp Thr Ile Phe Asn Leu Glu Asn Val Val Glu Tyr Thr Leu
    290                 295                 300

Cys Glu Gly Thr Lys Arg Lys Ile Asn Lys Gln Glu Lys Leu Thr Ser
305                 310                 315                 320

Val Asp Leu Ser Tyr Leu Ala Pro Arg Ile Gly Gly Phe Gly Ser Val
                325                 330                 335

Phe Arg Val Arg Asn Gly Thr Leu Glu Arg Gly Ser Thr Thr Tyr Ile
            340                 345                 350

Arg Ile Glu Val Glu Gly Pro Ile Val Asp Ser Leu Asn Gly Thr Asp
        355                 360                 365

Pro Arg Thr Asn Ala Ser Arg Val Phe Trp Asp Asp Trp Glu Leu Asp
    370                 375                 380

Gly Asn Ile Tyr Gln Gly Phe Asn Gly Val Tyr Lys Gly Lys Asp Gly
385                 390                 395                 400

Lys Ile His Ile Pro Leu Asn Met Ile Glu Ser Gly Ile Ile Asp Asn
                405                 410                 415

Glu Leu Gln His Ala Phe Gln Ala Asp Ile Ile Pro His Pro His Tyr
            420                 425                 430

Asp Asp Asp Glu Ile Arg Glu Asp Asp Ile Phe Phe Asp Asn Thr Gly
        435                 440                 445

Glu Asn Gly Asn Pro Val Asp Ala Val Val Glu Trp Val Ser Gly Trp
    450                 455                 460

Gly Thr Ser Leu Lys Phe Phe Gly Met Thr Leu Val Ala Leu Ile Met
465                 470                 475                 480

Ile Phe Leu Leu Ile Arg Cys Cys Val Ala Cys Thr Tyr Leu Met Lys
                485                 490                 495

Arg Ser Lys Arg Pro Ala Thr Glu Ser His Glu Met Arg Ser Leu Val
            500                 505                 510

<210>  21
<211>  1878
<212>  DNA
<213>  Artificial

<220>
<223>  Fusion protein SVCV-G + LT-B


<220>
<221>  CDS
<222>  (1)..(1857)

<400>  21
atg aac aag gtg aag tgt tat gtg ttg ttc act gca tta cta tcc tct      48
Met Asn Lys Val Lys Cys Tyr Val Leu Phe Thr Ala Leu Leu Ser Ser
1               5                   10                  15

cta tat gca cat ggt gct cct cag aca atc aca gaa ctt tgt tcc gaa      96
Leu Tyr Ala His Gly Ala Pro Gln Thr Ile Thr Glu Leu Cys Ser Glu
            20                  25                  30

tat cgc aac aca cag atc tac aca atc aat gac aag atc ctt tca tac      144
Tyr Arg Asn Thr Gln Ile Tyr Thr Ile Asn Asp Lys Ile Leu Ser Tyr
        35                  40                  45

act gaa tct atg gca ggc aaa aga gaa atg gtg atc atc aca ttc aag      192
Thr Glu Ser Met Ala Gly Lys Arg Glu Met Val Ile Ile Thr Phe Lys
    50                  55                  60

agc ggt gaa aca ttt caa gtc gaa gtc cca ggc agt caa cac att gat      240
Ser Gly Glu Thr Phe Gln Val Glu Val Pro Gly Ser Gln His Ile Asp
65                  70                  75                  80

tcc caa aag aag gcc att gaa agg atg aag gac acc ttg aga atc aca      288
Ser Gln Lys Lys Ala Ile Glu Arg Met Lys Asp Thr Leu Arg Ile Thr
                85                  90                  95

tat ctc aca gag acc aag att gac aag ttg tgt gtt tgg aac aac aag      336
Tyr Leu Thr Glu Thr Lys Ile Asp Lys Leu Cys Val Trp Asn Asn Lys
            100                 105                 110

act ccc aat tca att gct gca atc agc atg aag aac tac gct cca ggt      384
Thr Pro Asn Ser Ile Ala Ala Ile Ser Met Lys Asn Tyr Ala Pro Gly
        115                 120                 125

gtc gag ata ttt gtt cca tct ggt cga aat ata tca tgg cag cct gtg      432
Val Glu Ile Phe Val Pro Ser Gly Arg Asn Ile Ser Trp Gln Pro Val
    130                 135                 140

att cag cca ttt gat tat caa tgt cca atc cat gga aac cta ccc aac      480
Ile Gln Pro Phe Asp Tyr Gln Cys Pro Ile His Gly Asn Leu Pro Asn
145                 150                 155                 160

acg atg ggt ttg agt gcc acc aaa ttg aca ata aaa tct cca tct gta      528
Thr Met Gly Leu Ser Ala Thr Lys Leu Thr Ile Lys Ser Pro Ser Val
                165                 170                 175

ttc agt aca gat aaa gtg tct gga tgg atc tgc cat gca gct gaa tgg      576
Phe Ser Thr Asp Lys Val Ser Gly Trp Ile Cys His Ala Ala Glu Trp
            180                 185                 190

aaa aca act tgt gat tat aga tgg tat gga ccc caa tac atc acc cac      624
Lys Thr Thr Cys Asp Tyr Arg Trp Tyr Gly Pro Gln Tyr Ile Thr His
        195                 200                 205

agt att cat ccg atc agt cct acc ata gat gaa tgc agg aga atc att 672
Ser Ile His Pro Ile Ser Pro Thr Ile Asp Glu Cys Arg Arg Ile Ile
    210                 215                 220

caa agg att gca tca ggg act gat gaa gat ctg ggg ttt ccc cct caa 720
Gln Arg Ile Ala Ser Gly Thr Asp Glu Asp Leu Gly Phe Pro Pro Gln
225                 230                 235                 240

agt tgc gga tgg gca tct gtc aca acg gtg tca aat act aat tat aga 768
Ser Cys Gly Trp Ala Ser Val Thr Thr Val Ser Asn Thr Asn Tyr Arg
                245                 250                 255

gta gta ccc cat tct gtt cat tta gag cca tac gga gga cat tgg atc 816
Val Val Pro His Ser Val His Leu Glu Pro Tyr Gly Gly His Trp Ile
            260                 265                 270

gat cac gaa ttt aat ggg ggc gaa tgc aga gaa aaa gtg tgc gaa atg 864
Asp His Glu Phe Asn Gly Gly Glu Cys Arg Glu Lys Val Cys Glu Met
        275                 280                 285

aaa ggg aat cac tct att tgg atc aca gag gag act gta cag cac gag 912
Lys Gly Asn His Ser Ile Trp Ile Thr Glu Glu Thr Val Gln His Glu
    290                 295                 300

tgt gca aaa cat ata gag gaa gtt gaa gga atc atg tac ggg aat gtt 960
Cys Ala Lys His Ile Glu Glu Val Glu Gly Ile Met Tyr Gly Asn Val
305                 310                 315                 320

cca aga ggg gat gta atg tat gct aac aac ttc att ata gat aga cat 1008
Pro Arg Gly Asp Val Met Tyr Ala Asn Asn Phe Ile Ile Asp Arg His
                325                 330                 335

cat aga gta tac aga ttc ggg gga tct tgt cag atg aaa ttc tgt aat 1056
His Arg Val Tyr Arg Phe Gly Gly Ser Cys Gln Met Lys Phe Cys Asn
            340                 345                 350

aaa gat ggt ata aaa ttc gcg aga gga gac tgg gta gag aaa aca gcc 1104
Lys Asp Gly Ile Lys Phe Ala Arg Gly Asp Trp Val Glu Lys Thr Ala
        355                 360                 365

gga aca tta aca acg att cat gac aat gtg cct aaa tgt gtt gat gga 1152
Gly Thr Leu Thr Thr Ile His Asp Asn Val Pro Lys Cys Val Asp Gly
    370                 375                 380

acg ttg gtc tcc ggt cac cgc ccc gga tta gac ttg att gac aca atc 1200
Thr Leu Val Ser Gly His Arg Pro Gly Leu Asp Leu Ile Asp Thr Ile
385                 390                 395                 400

ttc aat ttg gaa aat gta gtg gaa tat act ttg tgt gaa ggg act aaa 1248
Phe Asn Leu Glu Asn Val Val Glu Tyr Thr Leu Cys Glu Gly Thr Lys
                405                 410                 415

agg aaa atc aat aaa caa gag aag ctg aca tca gtg gat ttg agc tat 1296
Arg Lys Ile Asn Lys Gln Glu Lys Leu Thr Ser Val Asp Leu Ser Tyr
            420                 425                 430

ttg gct cca aga atc gga ggg ttc gga tca gta ttt aga gtc aga aac 1344
Leu Ala Pro Arg Ile Gly Gly Phe Gly Ser Val Phe Arg Val Arg Asn
        435                 440                 445

gga aca tta gag aga ggg agc act act tat atc agg atc gaa gta gag 1392
Gly Thr Leu Glu Arg Gly Ser Thr Thr Tyr Ile Arg Ile Glu Val Glu
    450                 455                 460

gga cct att gtc gac tcg tta aat ggg aca gac cct aga act aac gcc 1440
Gly Pro Ile Val Asp Ser Leu Asn Gly Thr Asp Pro Arg Thr Asn Ala
465                 470                 475                 480

tca aga gta ttc tgg gat gac tgg gag tta gat ggc aat ata tac cag     1488
Ser Arg Val Phe Trp Asp Asp Trp Glu Leu Asp Gly Asn Ile Tyr Gln
                485                 490                 495

ggt ttt aat ggt gta tat aaa ggg aaa gat ggg aag atc cat atc cct     1536
Gly Phe Asn Gly Val Tyr Lys Gly Lys Asp Gly Lys Ile His Ile Pro
            500                 505                 510

tta aat atg ata gaa tca gga atc ata gat aat gag ctt caa cat gct     1584
Leu Asn Met Ile Glu Ser Gly Ile Ile Asp Asn Glu Leu Gln His Ala
        515                 520                 525

ttc caa gcc gat att atc cct cat cct cat tat gac gac gat gaa atc     1632
Phe Gln Ala Asp Ile Ile Pro His Pro His Tyr Asp Asp Asp Glu Ile
    530                 535                 540

cga gag gat gat ata ttt ttc gat aac act gga gaa aat gga aat cct     1680
Arg Glu Asp Asp Ile Phe Phe Asp Asn Thr Gly Glu Asn Gly Asn Pro
545                 550                 555                 560

gta gat gca gtg gta gaa tgg gtt agt gga tgg gga act agt cta aag     1728
Val Asp Ala Val Val Glu Trp Val Ser Gly Trp Gly Thr Ser Leu Lys
                565                 570                 575

ttc ttt ggc atg act ctg gtc gcc ctg atc atg atc ttt ctt ctc att     1776
Phe Phe Gly Met Thr Leu Val Ala Leu Ile Met Ile Phe Leu Leu Ile
            580                 585                 590

aga tgc tgt gtt gct tgc act tat ttg atg aag agg agt aaa cgg cct     1824
Arg Cys Cys Val Ala Cys Thr Tyr Leu Met Lys Arg Ser Lys Arg Pro
        595                 600                 605

gca aca gaa tca cac gaa atg cgg tct tta gtt tgatgacttg gatccgagct   1877
Ala Thr Glu Ser His Glu Met Arg Ser Leu Val
    610                 615

c                                                                   1878


<210>  22
<211>  619
<212>  PRT
<213>  Artificial

<220>
<223>  Synthetic Construct

<400>  22

Met Asn Lys Val Lys Cys Tyr Val Leu Phe Thr Ala Leu Leu Ser Ser
1               5                   10                  15


Leu Tyr Ala His Gly Ala Pro Gln Thr Ile Thr Glu Leu Cys Ser Glu
            20                  25                  30


Tyr Arg Asn Thr Gln Ile Tyr Thr Ile Asn Asp Lys Ile Leu Ser Tyr
        35                  40                  45


Thr Glu Ser Met Ala Gly Lys Arg Glu Met Val Ile Ile Thr Phe Lys
    50                  55                  60


Ser Gly Glu Thr Phe Gln Val Glu Val Pro Gly Ser Gln His Ile Asp
65                  70                  75                  80

Ser Gln Lys Lys Ala Ile Glu Arg Met Lys Asp Thr Leu Arg Ile Thr
                85                  90                  95

Tyr Leu Thr Glu Thr Lys Ile Asp Lys Leu Cys Val Trp Asn Asn Lys
            100                 105                 110

Thr Pro Asn Ser Ile Ala Ala Ile Ser Met Lys Asn Tyr Ala Pro Gly
        115                 120                 125

Val Glu Ile Phe Val Pro Ser Gly Arg Asn Ile Ser Trp Gln Pro Val
    130                 135                 140

Ile Gln Pro Phe Asp Tyr Gln Cys Pro Ile His Gly Asn Leu Pro Asn
145                 150                 155                 160

Thr Met Gly Leu Ser Ala Thr Lys Leu Thr Ile Lys Ser Pro Ser Val
                165                 170                 175

Phe Ser Thr Asp Lys Val Ser Gly Trp Ile Cys His Ala Ala Glu Trp
            180                 185                 190

Lys Thr Thr Cys Asp Tyr Arg Trp Tyr Gly Pro Gln Tyr Ile Thr His
        195                 200                 205

Ser Ile His Pro Ile Ser Pro Thr Ile Asp Glu Cys Arg Arg Ile Ile
    210                 215                 220

Gln Arg Ile Ala Ser Gly Thr Asp Glu Asp Leu Gly Phe Pro Pro Gln
225                 230                 235                 240

Ser Cys Gly Trp Ala Ser Val Thr Thr Val Ser Asn Thr Asn Tyr Arg
                245                 250                 255

Val Val Pro His Ser Val His Leu Glu Pro Tyr Gly Gly His Trp Ile
            260                 265                 270

Asp His Glu Phe Asn Gly Gly Glu Cys Arg Glu Lys Val Cys Glu Met
        275                 280                 285

Lys Gly Asn His Ser Ile Trp Ile Thr Glu Glu Thr Val Gln His Glu
    290                 295                 300

Cys Ala Lys His Ile Glu Glu Val Glu Gly Ile Met Tyr Gly Asn Val
305                 310                 315                 320

Pro Arg Gly Asp Val Met Tyr Ala Asn Asn Phe Ile Ile Asp Arg His
                325                 330                 335

His Arg Val Tyr Arg Phe Gly Gly Ser Cys Gln Met Lys Phe Cys Asn
            340                 345                 350

Lys Asp Gly Ile Lys Phe Ala Arg Gly Asp Trp Val Glu Lys Thr Ala
        355                 360                 365

Gly Thr Leu Thr Thr Ile His Asp Asn Val Pro Lys Cys Val Asp Gly
    370                 375                 380

Thr Leu Val Ser Gly His Arg Pro Gly Leu Asp Leu Ile Asp Thr Ile
385                 390                 395                 400

Phe Asn Leu Glu Asn Val Val Glu Tyr Thr Leu Cys Glu Gly Thr Lys
                405                 410                 415

Arg Lys Ile Asn Lys Gln Glu Lys Leu Thr Ser Val Asp Leu Ser Tyr
            420                 425                 430

Leu Ala Pro Arg Ile Gly Gly Phe Gly Ser Val Phe Arg Val Arg Asn
        435                 440                 445

Gly Thr Leu Glu Arg Gly Ser Thr Thr Tyr Ile Arg Ile Glu Val Glu
    450                 455                 460

Gly Pro Ile Val Asp Ser Leu Asn Gly Thr Asp Pro Arg Thr Asn Ala
465                 470                 475                 480

Ser Arg Val Phe Trp Asp Asp Trp Glu Leu Asp Gly Asn Ile Tyr Gln
                485                 490                 495

Gly Phe Asn Gly Val Tyr Lys Gly Lys Asp Gly Lys Ile His Ile Pro
            500                 505                 510

Leu Asn Met Ile Glu Ser Gly Ile Ile Asp Asn Glu Leu Gln His Ala
        515                 520                 525

Phe Gln Ala Asp Ile Ile Pro His Pro His Tyr Asp Asp Asp Glu Ile
    530                 535                 540

Arg Glu Asp Asp Ile Phe Phe Asp Asn Thr Gly Glu Asn Gly Asn Pro
545                 550                 555                 560

Val Asp Ala Val Val Glu Trp Val Ser Gly Trp Gly Thr Ser Leu Lys
                565                 570                 575

Phe Phe Gly Met Thr Leu Val Ala Leu Ile Met Ile Phe Leu Leu Ile
            580                 585                 590

Arg Cys Cys Val Ala Cys Thr Tyr Leu Met Lys Arg Ser Lys Arg Pro
        595                 600                 605

Ala Thr Glu Ser His Glu Met Arg Ser Leu Val
    610                 615